# EUROPEAN PATENT APPLICATION

(11) **EP 4 733 765 A2**
(43) Date of publication of application: **29.04.2026**
(21) Application number: 25226931.1
(22) Date of filing: 19.02.2021
(51) Int. Cl.: G01N 33/569

(54) **COMPOSITION FOR IMPROVING PULMONARY HYPERTENSION, METHOD FOR PREDICITING PROGNOSIS OF PULMONARY HYPERTENSION, METHOD FOR ASSISTING DETERMINATION OF SEVERITY OF PULMONARY HYPERTENSION, AND METHOD FOR ASSISTING DIAGNOSIS OF PULMONARY HYPERTENSION**

(30) Priority: 21.02.2020 JP 2020028865
(62) Divisional of application: 21757760.0
(71) Applicant: Nakaoka, Yoshikazu, Suita-shi, Osaka, 564-8565 (JP); Asano, Ryotaro, Suita-shi, Osaka, 564-8565 (JP); Ogo, Takeshi, Suita-shi, Osaka, 564-8565 (JP)
(72) Inventor: Nakaoka, Yoshikazu, Suita-shi, Osaka, 564-8565 (JP); Asano, Ryotaro, Suita-shi, Osaka, 564-8565 (JP); Ogo, Takeshi, Suita-shi, Osaka, 564-8565 (JP)
(74) Representative: Kraus & Lederer PartGmbB

(57) **Abstract**

The present invention provides (1) a composition for improving pulmonary hypertension, comprising at least one substance capable of normalizing gut microbiota in a patient with pulmonary hypertension as an active ingredient; (2) a method for predicting the prognosis of a patient with pulmonary hypertension, or a method for assisting the determination of the severity of a patient with pulmonary hypertension, the method comprising detecting one or more types of bacteria selected from bacteria belonging to the family Micrococcaceae, Streptococcaceae, Pasteurellaceae, Veillonellaceae or Lactobacillaceae in gut microbiota in the patient with pulmonary hypertension; and (3) a method for assisting the diagnosis of pulmonary hypertension, the method comprising comparing the IgA level in feces of a subject to that of a healthy subject.

## Description

### TECHNICAL FIELD

The present invention relates to a composition for improving pulmonary hypertension, a method for predicting the prognosis of pulmonary hypertension, a method for assisting the determination of the severity of pulmonary hypertension, and a method for assisting the diagnosis of pulmonary hypertension.

### BACKGROUND ART

Pulmonary hypertension is an intractable disease with a poor prognosis, which is characterized by pulmonary arterial remodeling, including stenosis and occlusion, leading to elevation of the pulmonary arterial pressure and eventually right-sided heart failure. Pulmonary hypertension is classified into five groups based on the underlying cause of the disease (see Table 1). Some of the pulmonary hypertension diseases have been designated as intractable diseases by the Ministry of Health, Labour and Welfare, including pulmonary arterial hypertension, which is caused by pulmonary artery remodeling such as pulmonary arterial medial hypertrophy and intimal proliferation lesions; and chronic thromboembolic pulmonary hypertension, which is caused by chronic thrombus occlusion in the pulmonary artery.

Currently, several classes of drugs are available to treat pulmonary hypertension in Japan, including endothelin receptor antagonists, such as bosentan, ambrisentan and macitentan; phosphodiesterases (PDE) 5 inhibitors, such as sildenafil and tadalafil; soluble guanylate cyclase (sGC) stimulators, such as riociguat; and prostaglandin I2 and its derivatives, such as epoprostenol, selexipag, iloprost and treprostinil. These therapeutic drugs are clinically used to treat pulmonary hypertension. However, some patients are refractory to such treatment, and the prognosis of these patients is very poor. Therefore, there is still a need for the development of a novel therapy or a biomarker for pulmonary hypertension.

BMPR2 signaling-related genes are known genetic predispositions for pulmonary hypertension, but the genetic penetrance is as low as about 20%. Inflammation or exposure to exogenous chemicals is believed to be critical for the development of pulmonary hypertension. The inventors reported that interleukin-6 (IL-6)/Th17 cells/IL-21-signaling axis is critical in the pathogenesis of pulmonary hypertension (non-patent literature 1). Th17 cells playing an important role in the signaling axis are most abundant in the intestinal tract, and interaction between Th17 cells and gut microbiota is important for inducing the differentiation of Th17 cells. Thus, the role of gut microbiota in the phenotypes of pulmonary hypertension was investigated in this study.

**Table 1**

| **Group 1. Pulmonary arterial hypertension (PAH)** | | **Group 2. Pulmonary hypertension due to left heart disease** |
|---|---|---|
| **1) Idiopathic PAH (IPAH)** | | 1) Systolic dysfunction |
| **2) Heritable PAH (HPAH)** | | 2) Diastolic dysfunction |
| | 1. BMPR2 | 3) Valvular disease |
| | 2. ALK1, endoglin, SMAD9, CAV1, KCNK3 | 4) Congenital or acquired left ventricular inflow and outflow obstruction |
| | 3. Unknown | |
| **3) Drugs and toxins induced PAH** | | **Group 3. Pulmonary hypertension due to lung diseases and/or hypoxia** |
| **4) Disease-associated PAH (APAH)** | | |
| | 1. Connective tissue diseases | 1) Chronic obstructive lung disease |
| | 2. HIV infection | 2) Interstitial lung disease |
| | 3. Portal pulmonary hypertension | 3) Other lung diseases with mixed restrictive and obstructive pattern |
| | 4. Congenital systemic-to-pulmonary shunt | |
| | 5. Schistosomiasis | 4) Sleep-disordered breathing |
| | | 5) Alveolar hypoventilation |
| | | 6) Chronic exposure to high altitude |
| | | 7) Developmental abnormalities |
| | | **Group 4. Chronic thromboembolic pulmonary hypertension (CTEPH)** |
| | | **Group 5. Pulmonary hypertension with unclear and/or multifactorial mechanisms** |
| 1'. Pulmonary veno-occlusive disease (PVOD) and/or pulmonary capillary hemangiomatosis (PCH) | | 1) Hematologic disorders (chronic hemolytic anemia, myeloproliferative disorders, splenectomy) |
| 1". Neonatal persistent pulmonary hypertension | | 2) Systemic disorders (sarcoidosis, pulmonary Langerhans cell histiocytosis, lymphangioleiomyomatosis, neurofibromatosis, vasculitis) |
| | | 3) Metabolic disorders (glycogen storage disease, Gaucher disease, thyroid disorders) |
| | | 4) Others (obstruction by tumors, fibrosingmediastinitis, chronic renal failure on dialysis, segmental PH) |

### CITATION LIST

### NON-PATENT LITERATURE

Non-patent literature 1: Hashimoto-Kataoka T, et al. Proc Natl Acad Sci USA 112, E2677-2686 (2015).

### SUMMARY OF INVENTION

### TECHNICAL PROBLEM

An object of the present invention is to provide a composition that improves pulmonary hypertension by acting on gut microbiota in a patient with pulmonary hypertension. Another object of the present invention is to provide a method for predicting the prognosis of a patient with pulmonary hypertension and a method for assisting the determination of the severity of a patient with pulmonary hypertension, the method comprising detecting specific gut bacteria in the patient with pulmonary hypertension. A further object of the present invention is to provide a method for assisting the diagnosis of pulmonary hypertension using the feces of a subject.

### SOLUTION TO PROBLEM

The present invention was made to solve the above problems and includes the following.
(1) A composition for improving pulmonary hypertension, comprising at least one substance capable of normalizing gut microbiota in a patient with pulmonary hypertension as an active ingredient.
(2) The composition according to the above (1), wherein the substance is a substance capable of reducing gut bacteria that are increased in a patient with pulmonary hypertension compared to those in a healthy subject.
(3) The composition according to the above (2), wherein the gut bacteria that are increased in a patient with pulmonary hypertension are bacteria belonging to the family Streptococcaceae, Micrococcaceae, Veillonellaceae, Pasteurellaceae, Clostridiaceae or Sutterellaceae.
(3-1) The composition according to the above (2), wherein the gut bacteria that are increased in a patient with pulmonary hypertension are bacteria belonging to the family Streptococcaceae, Micrococcaceae, Veillonellaceae, Pasteurellaceae, Fusobacteriaceae, Lactobacillaceae, Enterobacteriaceae, Coriobacteriaceae or Sutterellaceae.
(4) The composition according to the above (2), wherein the gut bacteria that are increased in a patient with pulmonary hypertension are bacteria belonging to the genus *Actinomyces, Rothia, Citrobacter, Veillonella, Escherichia, Gemella, Granulicatella, Atopobium, Clostridium, Enterobacter, Streptococcus, Abiotrophia, Klebsiella, Cronobacter, Shigella, Salmonella, Sutterella* or *Haemophilus.*
(4-1) The composition according to the above (2), wherein the gut bacteria that are increased in a patient with pulmonary hypertension are bacteria belonging to the genus *Actinomyces, Rothia, Citrobacter, Veillonella, Escherichia, Gemella, Granulicatella, Atopobium, Enterobacter, Streptococcus, Abiotrophia, Klebsiella, Cronobacter, Shigella, Salmonella, Sutterella, Lachnoclostridium, Fusobacterium, Lactobacillus, Erysipelatoclostridium, Collinsella, Tyzzerella* or *Haemophilus.*
(5) The composition according to the above (2), wherein the gut bacteria that are increased in a patient with pulmonary hypertension are *Streptococcus infantis, Streptococcus parasanguinis, Ruminococcus gnavus, Clostridium bolteae, Sutterella wadsworthensis, Klebsiella pneumoniae, Rothia mucilaginosa, Streptococcus mitis, Streptococcus oralis, Streptococcus pneumoniae, Streptococcus salivarius, Haemophilus parainfluenzae, Veillonella parvula* or *Veillonella* unclassified.
(6) The composition according to the above (1) or (2), wherein the substance is an agent having antibacterial activity against normal flora bacteria in an oral cavity.
(7) The composition according to the above (6), wherein the agent having antibacterial activity against normal flora bacteria in an oral cavity is a penicillin, cephem, penem, carbapenem, macrolide, lincomycin, ketolide, newquinolone, glycopeptide, streptogramin, tetracycline, chloramphenicol, quinolone, peptide, aminoglycoside, monobactam, nitroimidazole or fosfomycin antibiotic.
(8) The composition according to the above (1) or (2), wherein the substance is a phage exhibiting bacteriolytic activity against gut bacteria that are increased in a patient with pulmonary hypertension compared to those in a healthy subject.
(9) The composition according to the above (1), wherein the substance is a substance capable of increasing gut bacteria that are decreased in a patient with pulmonary hypertension compared to those in a healthy subject.
(10) The composition according to the above (9), wherein the gut bacteria that are decreased in a patient with pulmonary hypertension are bacteria belonging to the family Rikenellaceae, Coriobacteriaceae, Ruminococcaceae, Alcaligenaceae, Bacteroidaceae, Eubacteriaceae, Lachnospiraceae, Desulfovibrionaceae, Bifidobacteriaceae, Sutterellaceae, Lachnospiraceae or Porphyromonadaceae.
(10-1) The composition according to the above (9), wherein the gut bacteria that are decreased in a patient with pulmonary hypertension are bacteria belonging to the family Rikenellaceae, Ruminococcaceae, Alcaligenaceae, Bacteroidaceae, Eubacteriaceae, Lachnospiraceae, Desulfovibrionaceae, Bifidobacteriaceae, Sutterellaceae, Prevotellaceae, Clostridiaceae, Eggerthellaceae or Porphyromonadaceae.
(11) The composition according to the above (9), wherein the gut bacteria that are increased in a patient with pulmonary hypertension are bacteria belonging to the genus *Butyricimonas, Alistipes, Ruminococcus, Adlercreutzia, Acidaminococcus, Sutterella, Oscillospira, Rikenella, Lachnospira, Collinsella, Holdemania, Eubacterium, Subdoligranulum, Bilophila, Bifidobacterium, Parasutterella, Roseburia*, *Faecalibacterium, Parabacteroides* or *Bacteroides.*
(11-1) The composition according to the above (9), wherein the gut bacteria that are decreased in a patient with pulmonary hypertension are bacteria belonging to the genus *Butyricimonas, Alistipes, Ruminococcus, Adlercreutzia, Acidaminococcus, Sutterella, Oscillospira, Rikenella, Lachnospira, Holdemania, Eubacterium, Subdoligranulum, Bilophila, Bifidobacterium, Parasutterella, Roseburia, Faecalibacterium, Parabacteroides, Megamonas, Phascolarctobacterium, Agathobacter, Hydrogenoanaerobacterium, Blautia, Ruminiclostridium, Paraprevotella, Acetanaerobacterium, Dorea, Fusicatenibacter, Coprococcus, Gordonibacter, Coprobacter, Oscillibacter, Anaerostipes* or *Bacteroides.*
(12) The composition according to the above (9), wherein the gut bacteria that are decreased in a patient with pulmonary hypertension are *Eubacterium hallii, Bilophila* unclassified, *Bifidobacterium adolescentis, Bifidobacterium pseudocatenulatum, Parasutterella excrementihominis, Roseburia hominis, Alistipes onderdonkii, Faecalibacterium prausnitzii, Eubacterium ventriosum, Roseburia intestinalis, Parabacteroides johnsonii, Bacteroides cellulosilyticus, Bacteroides uniformis*, *Eubacterium eligens, Alistipes* sp. AP11, Bacteroidales bacterium ph8, *Subdoligranulum* sp. 4_3_54A2FAA or Lachnospiraceae bacterium 1_1_57FAA.
(13) The composition according to the above (9), wherein the composition comprises one or more types of bacteria selected from the bacteria according to any one of the above (10) to (12) as an active ingredient.
(13-1) The composition according to the above (9), wherein the composition comprises one or more types of bacteria selected from the bacteria according to any one of the above (10-1) to (12) as an active ingredient.
(14) A composition for improving pulmonary hypertension, comprising, as an active ingredient, a substance capable of inhibiting at least one pathway selected from the group consisting of a mevalonate pathway, a mannosylglycerate synthesis pathway, a methylglyoxal degradation pathway, a D-glucarate degradation pathway, a TCA cycle VIII pathway, a heme biosynthesis pathway and a nitrate metabolism pathway.
(15) The composition according to the above (14), wherein the active ingredient is a substance capable of inhibiting a mevalonate pathway, and wherein the substance is an HMG-CoA reductase inhibitor.
(16) A method for predicting the prognosis of a patient with pulmonary hypertension, the method comprising detecting one or more types of bacteria selected from bacteria belonging to the family Micrococcaceae, Streptococcaceae or Pasteurellaceae in gut microbiota in the patient with pulmonary hypertension.
(16-1) A method for predicting the prognosis of a patient with pulmonary hypertension, the method comprising detecting one or more types of bacteria selected from bacteria belonging to the family Micrococcaceae, Streptococcaceae, Pasteurellaceae, Veillonellaceae or Lactobacillaceae in gut microbiota in the patient with pulmonary hypertension.
(17) A method for assisting the determination of the severity of a patient with pulmonary hypertension, the method comprising detecting one or more types of bacteria selected from bacteria belonging to the family Micrococcaceae, Streptococcaceae or Pasteurellaceae in gut microbiota in the patient with pulmonary hypertension.
(17-1) A method for assisting the determination of the severity of a patient with pulmonary hypertension, the method comprising detecting one or more types of bacteria selected from bacteria belonging to the family Micrococcaceae, Streptococcaceae, Pasteurellaceae, Veillonellaceae or Lactobacillaceae in gut microbiota in the patient with pulmonary hypertension.
(18) The method according to the above (17), wherein the Micrococcaceae bacteria are *Rothia* bacteria, and wherein the Streptococcaceae bacteria are *Streptococcus* bacteria.
(18-1) The method according to the above (17-1), wherein the Micrococcaceae bacteria are *Rothia* bacteria, wherein the Streptococcaceae bacteria are *Streptococcus* bacteria, and wherein the Veillonellaceae bacteria are *Veillonella* bacteria.
(19) A method for assisting the diagnosis of pulmonary hypertension, the method comprising measuring an IgA level in feces of a subject, and comparing the measured IgA level to that of a healthy subject.
(20) A composition for improving pulmonary hypertension, comprising a short-chain fatty acid or a salt thereof as an active ingredient.
(21) A method for assisting the diagnosis of portal hypertension-associated pulmonary hypertension, the method comprising detecting one or more types of bacteria selected from *Bifidobacterium* bacteria, *Dorea* bacteria and *Blautia* bacteria in gut microbiota in a subject.
(22) A method for assisting the diagnosis of chronic thromboembolic pulmonary hypertension, the method comprising detecting *Klebsiella* bacteria in gut microbiota in a subject.

### ADVANTAGEOUS EFFECTS OF INVENTION

The present invention provides a composition that improves pulmonary hypertension by acting on gut microbiota in a patient with pulmonary hypertension. The present invention also provides a method for predicting the prognosis of a patient with pulmonary hypertension and a method for assisting the determination of the severity of a patient with pulmonary hypertension, the method comprising detecting specific gut bacteria in the patient with pulmonary hypertension. The present invention also provides a method for assisting the diagnosis of pulmonary hypertension using the feces of a subject.

### BRIEF DESCRIPTION OF DRAWINGS

Fig. 1 shows the procedures of preparation of rat models of pulmonary hypertension. The upper, middle and lower rows show the procedures of preparation of rat models of pulmonary hypertension induced by hypoxia exposure, monocrotaline administration, and Sugen 5416/hypoxia/normoxia exposure, respectively.
Fig. 2 shows the composition of the gut microbiota at the familial level analyzed by 16S rRNA metagenomic analysis of DNA extracted from the feces of three types of rat models of pulmonary hypertension prepared by subjecting wild-type rats to hypoxia (Hx), monocrotaline (MCT) administration or Sugen 5416/hypoxia/normoxia (SuHx); DNA extracted from the feces of rats deficient in the aryl hydrocarbon receptor (AHR) subjected to Sugen 5416/hypoxia/normoxia (SuHx); and DNA extracted from the feces of non-treated rats (control group).
Fig. 3(A) is a chart showing the abundance of S24-7 in the gut microbiota in the control group, the Hx group, the MCT group and the SuHx group. Fig. 3(B) shows the abundance of Lachnospiraceae in the gut microbiota in the control group, the Hx group, the MCT group and the SuHx group.
Figs. 4(A) and 4(B) are charts showing the changes in the phenotypes of pulmonary hypertension of the rat model of hypoxia-induced pulmonary hypertension (Hx) after receiving an antibiotic cocktail.
Figs. 5(A) and 5(B) are charts showing the changes in the phenotypes of pulmonary hypertension of the rat model of monocrotaline-induced pulmonary hypertension (MCT) after receiving an antibiotic cocktail.
Figs. 6(A) and 6(B) are charts showing the changes in the phenotypes of pulmonary hypertension of the rat model of Sugen 5416/hypoxia/normoxia-induced pulmonary hypertension (SuHx) after receiving an antibiotic cocktail.
Fig. 7 shows the composition of the gut microbiota analyzed at the familial level by 16S rRNA metagenomic analysis of DNA in fecal samples of patients with pulmonary hypertension (PH) and healthy subjects.
Figs. 8(A) and 8(B) show the evaluation of the alpha diversity of the gut microbiota in PH patients and healthy subjects. Fig. 8(A) shows the Shannon index and Fig. 8(B) shows the Chao index.
Fig. 9 shows the evaluation of the beta diversity of the gut microbiota in PH patients and healthy subjects as assessed by principal component analysis.
Figs. 10(A) to 10(C) are charts showing the abundance of the gut bacteria that were increased in PH patients. The abundance of the gut bacteria was plotted for individual healthy subjects and PH patients and compared between the healthy subject group and the PH patient group. Fig. 10(A) shows the results of Streptococcaceae. Fig. 10(B) shows the results of Micrococcaceae. Fig. 10(C) shows the results of Pasteurellaceae.
Figs. 11(A) to 11(C) are charts showing the relation between the changes in the gut bacteria and the severity of pulmonary hypertension in PH patients. Fig. 11(A) shows the results of Micrococcaceae. Fig. 11(B) shows the results of Streptococcaceae. Fig. 11(C) shows the results of Pasteurellaceae.
Figs. 12(A) to 12(C) are charts showing the relation between the changes in the gut bacteria and the prognosis of pulmonary hypertension in PH patients. Fig. 12(A) shows the results of Micrococcaceae. Fig. 12(B) shows the results of Streptococcaceae. Fig. 12(C) shows the results of Pasteurellaceae.

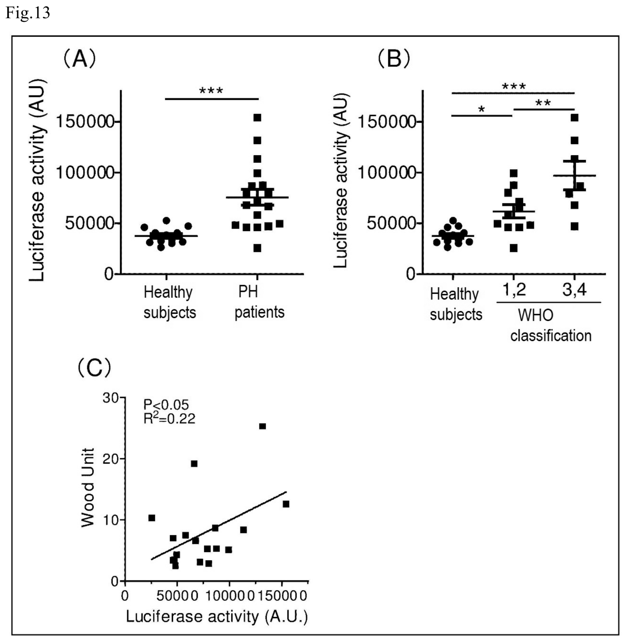

Figs. 13(A) to 13(C) are charts showing the relation between the serum aryl hydrocarbon receptor (AHR) activity and the severity of pulmonary hypertension in PH patients. Fig. 13(A) is a chart showing the measurement results of the serum AHR activity in PH patients and healthy subjects. Fig. 13(B) is a chart showing the measurement results of the serum AHR activity in a patient group with severity 1-2 according to the WHO classification, a patient group with severity 3-4 according to the WHO classification and healthy subjects. Fig. 13(C) is a chart showing the correlation between the pulmonary vascular resistance (PVR) and the serum AHR activity in PH patients.
Fig. 14 is a chart showing the relation between the changes in the gut bacteria and the serum AHR activity.
Figs. 15(A) to 15(P) are charts showing the gut bacteria that were increased in PH patients as compared to those in healthy subjects at the genus level. The charts show the bacteria belonging to the genus (A) *Actinomyces, (B) Rothia, (C) Citrobacter, (D) Veillonella,* (E) *Escherichia,* (F) *Gemella,* (G) *Granulicatella,* (H) *Atopobium,* (I) *Clostridium,* (J) *Enterobacter,* (K) *Streptococcus,* (L) *Abiotrophia,* (M) *Klebsiella,* (N) *Cronobacter,* (O) *Shigella* or (P) *Salmonella.*
Figs. 16(A) to 16(C) are charts showing the abundance of the gut bacteria that were decreased in PH patients. The abundance of the gut bacteria was plotted for individual healthy subjects and PH patients and compared between the healthy subject group and the PH patient group. Fig. 16(A) shows the results of Rikenellaceae. Fig. 16(B) shows the results of Coriobacteriaceae. Fig. 16(C) shows the results of Alcaligenaceae.
Figs. 17(A) to 17(K) are charts showing the gut bacteria that were decreased in PH patients as compared to those in healthy subjects at the genus level. The charts show the bacteria belonging to the genus (A) *Butyricimonas,* (B) *Alistipes,* (C) *Ruminococcus, (D) Adlercreutzia, (E) Acidaminococcus, (F) Sutterella, (G) Oscillospira, (H) Rikenella,* (I) *Lachnospira,* (J) *Collinsella* or (K) *Holdemania.*
Fig. 18 shows the results of whole metagenomic analysis of DNA in fecal samples of PH patients and healthy subjects.
Fig. 19 shows the results of analysis of the functional metabolic profile of the gut microbiota using the data generated by the whole metagenomic analysis.
Figs. 20(A) and 20(B) are charts showing the measurement results of the IgA levels in feces. Fig. 20(A) shows the IgA levels in the fecal samples of rat models of pulmonary hypertension. Fig. 20(B) shows the IgA levels in the fecal samples of PH patients.
Fig. 21 shows the results of PLS-DA analysis of the gut microbiota in PH patients and healthy subjects.
Figs. 22(A) to 22(C) show the evaluation of the alpha diversity of the gut microbiota in PH patients and healthy subjects. Fig. 22(A) shows the Faith's phylogenetic diversity. Fig. 22(B) shows the Shannon index. Fig. 22(C) shows the observed OTUs.
Fig. 23 shows the gut bacteria that were statistically significantly increased or decreased (at the genus level) in PH patients as determined using a LDA score of 3.0 or more as a cut-off value.
Fig. 24 is a volcano plot generated using significant differences in changes (-Log₁₀ (p-value)) against the fold change (Effect size) in PH patients. The dot sizes reflect the relative abundance of the gut bacteria at the genus level that were present in healthy subjects and the PH patients.
Fig. 25 is a cladogram generated based on the LDA scores and the volcano plot to depict the gut bacteria that were significantly changed at the genus level.

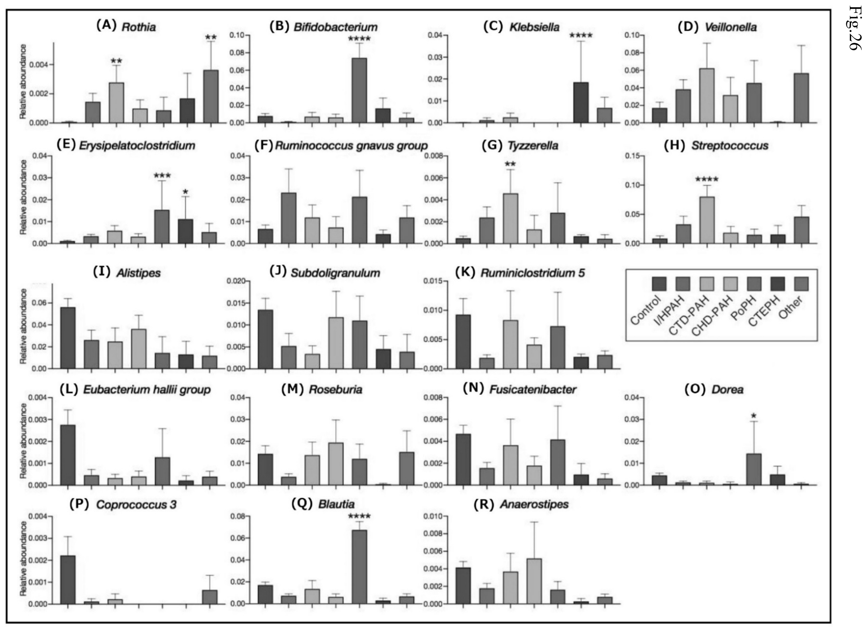

Figs. 26(A) to 26(R) are charts showing the frequencies of 18 different bacteria (at the genus level) that were increased or decreased when compared between the subgroups of pulmonary hypertension. The frequencies are shown respectively in (A) *Rothia,* (B) *Bifidobacterium,* (C) *Klebsiella,* (D) *Veillonella,* (E) *Erysipelatoclostridium,* (F) *Ruminococcus gnavus* group, (G) *Tyzzerella,* (H) *Streptococcus,* (I) *Alistipes,* (J) *Subdoligranulum,* (K) *Ruminiclostridium* 5, (L) *Eubacterium hallii* group, (M) *Roseburia,* (N) *Fusicatenibacter,* (O) *Dorea,* (P) *Coprococcus,* (Q) *Blautia* and (R) *Anaerostipes.*
Fig. 27 is a chart showing the ranking of the gut bacteria that are associated with the occurrence of pulmonary hypertension-related events (composite events including right-sided heart failure, lung transplantation, and death) as determined by random forest analysis.
Figs. 28(A) to 28(C) are charts showing the analysis of the relation between the frequencies of the gut bacteria and the severity of pulmonary hypertension (according to the WHO Functional Classification of Pulmonary Hypertension) in PH patients divided into two groups using a cut-off value that most accurately predicts the occurrence of the composite events as calculated by ROC analysis. The relation between the frequencies of the gut bacteria and the severity of pulmonary hypertension is shown in Figs. 28(A) *Rothia,* (B) *Veillonella* and (C) *Streptococcus.*
Figs. 29(A) to 29(C) are charts showing the comparison of the event-free survival using the Kaplan-Meier test in PH patients divided into two groups based on the prediction of the occurrence of the pulmonary hypertension-related events using cut-off values for *Rothia, Veillonella* and *Streptococcus.* The event-free survival is shown in Figs. 29(A) *Rothia,* (B) *Veillonella* and (C) *Streptococcus.*
Fig. 30 shows the network analysis of the gut bacteria using a correlation coefficient (r) of > 2 between the relative abundance of the gut bacteria (at the genus level) and the serum IL-6 levels calculated using a round-robin approach.
Figs. 31(A) and 31(B) are charts showing the evaluation of the phenotypes of pulmonary hypertension in a gnotobiotic rat model of pulmonary hypertension receiving fecal transplantation from healthy subjects and a gnotobiotic rat model of pulmonary hypertension receiving fecal transplantation from PH patients. Fig. 31(A) shows the right ventricular systolic pressure, and Fig. 31(B) shows the ratio of RV to LV weight (the right ventricular hypertrophy).
Figs. 32(A) to 32(C) show the measurement results of the acetic acid, propionic acid and butyric acid levels in the feces of healthy subjects and PH patients.
Figs. 33(A) and 33(B) show charts showing the evaluation of the phenotypes of pulmonary hypertension in a rat model of pulmonary hypertension that were given butyrate-containing water. Fig. 33(A) shows the right ventricular systolic pressure, and Fig. 33(B) shows the ratio of RV to LV weight (the right ventricular hypertrophy).

### DESCRIPTION OF EMBODIMENTS

### Composition for improving pulmonary hypertension

The present invention provides a composition for improving pulmonary hypertension, comprising at least one substance capable of normalizing gut microbiota in a patient with pulmonary hypertension as an active ingredient (hereinafter referred to as the composition of the present invention). The inventors performed metagenomic analysis of DNA in fecal samples of patients with pulmonary hypertension and healthy subjects and compared the composition of the gut microbiota between the patients and the healthy subjects. The inventors found that some gut bacteria were increased or decreased in the patients with pulmonary hypertension compared to those in the healthy subjects. Based on this finding, the inventors assumed that the severity of pulmonary hypertension could be improved by normalizing the composition of the gut microbiota in patients with pulmonary hypertension, i.e., altering the composition of the gut microbiota in patients with pulmonary hypertension to make it closer to that in healthy subjects.

The gut bacteria that are increased at the familial level in a patient with pulmonary hypertension compared to those in a healthy subject include, for example, bacteria belonging to the family Streptococcaceae, Micrococcaceae, Veillonellaceae, Pasteurellaceae, Fusobacteriaceae, Lactobacillaceae, Enterobacteriaceae, Coriobacteriaceae, Sutterellaceae, etc.

The gut bacteria that are increased at the genus level in a patient with pulmonary hypertension compared to those in a healthy subject include, for example, bacteria belonging to the genus *Actinomyces, Rothia, Citrobacter, Veillonella, Escherichia, Gemella, Granulicatella, Atopobium, Enterobacter, Streptococcus, Abiotrophia, Klebsiella, Cronobacter, Shigella, Salmonella, Sutterella, Lachnoclostridium, Fusobacterium, Lactobacillus, Erysipelatoclostridium, Collinsella, Tyzzerella, Haemophilus,* etc.

The gut bacteria that are increased at the species level in a patient with pulmonary hypertension compared to those in a healthy subject include, for example, *Streptococcus infantis*, *Streptococcus parasanguinis, Ruminococcus gnavus, Clostridium bolteae, Sutterella wadsworthensis, Klebsiella pneumoniae, Rothia mucilaginosa, Streptococcus mitis, Streptococcus oralis, Streptococcus pneumoniae, Streptococcus salivarius, Haemophilus parainfluenzae, Veillonella parvula, Veillonella* unclassified, etc.

The gut bacteria that are decreased at the familial level in a patient with pulmonary hypertension compared to those in a healthy subject include, for example, bacteria belonging to the family Rikenellaceae, Ruminococcaceae, Alcaligenaceae, Bacteroidaceae, Eubacteriaceae, Lachnospiraceae, Desulfovibrionaceae, Bifidobacteriaceae, Sutterellaceae, Prevotellaceae, Clostridiaceae, Eggerthellaceae, Porphyromonadaceae, etc.

The gut bacteria that are decreased at the genus level in a patient with pulmonary hypertension compared to those in a healthy subject include, for example, bacteria belonging to the genus *Butyricimonas, Alistipes, Ruminococcus, Adlercreutzia, Acidaminococcus, Sutterella, Oscillospira, Rikenella, Lachnospira, Holdemania, Eubacterium, Subdoligranulum, Bilophila, Bifidobacterium, Parasutterella, Roseburia, Faecalibacterium, Parabacteroides, Megamonas, Phascolarctobacterium, Agathobacter, Hydrogenoanaerobacterium, Blautia, Ruminiclostridium, Paraprevotella, Acetanaerobacterium, Dorea, Fusicatenibacter, Coprococcus, Gordonibacter, Coprobacter, Oscillibacter, Anaerostipes, Bacteroides,* etc.

The gut bacteria that are decreased at the species level in a patient with pulmonary hypertension compared to those in a healthy subject include, for example, *Eubacterium hallii, Bilophila* unclassified, *Bifidobacterium adolescentis, Bifidobacterium pseudocatenulatum, Parasutterella excrementihominis, Roseburia hominis, Alistipes onderdonkii, Faecalibacterium prausnitzii, Eubacterium ventriosum, Roseburia intestinalis, Parabacteroides johnsonii, Bacteroides cellulosilyticus, Bacteroides uniformis*, *Eubacterium eligens, Alistipes* sp. AP11, Bacteroidales bacterium ph8, *Subdoligranulum* sp. 4_3_54A2FAA, Lachnospiraceae bacterium 1_1_57FAA, etc.

The composition of the present invention comprises at least one substance capable of normalizing gut microbiota in a patient with pulmonary hypertension as an active ingredient. The pulmonary hypertension to be treated may be pulmonary hypertension classified into Group 1, Group 2, Group 3, Group 4 or Group 5 according to Table 1.

The active ingredient of the composition of the present invention may be a substance capable of reducing gut bacteria that are increased in a patient with pulmonary hypertension compared to those in a healthy subject. The substance may be an agent having antibacterial activity against normal flora bacteria in an oral cavity. The gut bacteria that are increased in a patient with pulmonary hypertension compared to those in a healthy subject include many types of normal flora bacteria in the oral cavity. Therefore, an agent having antibacterial activity against normal flora bacteria in the oral cavity is useful as an active ingredient of the composition of the present invention. Examples of the normal flora bacteria in the oral cavity include *Streptococcus infantis*, *Streptococcus parasanguinis, Klebsiella pneumoniae, Rothia mucilaginosa, Streptococcus mitis, Streptococcus oralis, Streptococcus pneumoniae, Streptococcus salivarius, Veillonella parvula, Veillonella* unclassified, etc.

The agent having antibacterial activity against normal flora bacteria in the oral cavity may be any one of penicillin antibacterial compounds, such as methicillin, oxacillin, nafcillin, cloxacillin, dicloxacillin, flucloxacillin, temocillin, amoxicillin, piperacillin, talampicillin, bacampicillin, ampicillin, ticarcillin, benzylpenicillin and carbenicillin; cephem antibacterial compounds, such as cephalothin, cefazolin, cefotiam, cefmetazole, cefotaxime, cefmenoxime, cefodizime, ceftriaxone, ceftazidime, cefoperazone, cefminox, latamoxef, flomoxef, cefpirome, cefepime, cefozopran and cefalexin; penem antibacterial compounds, such as faropenem; carbapenem antibacterial compounds, such as imipenem, panipenem, meropenem, biapenem, doripenem, ertapenem and tebipenem; macrolide antibacterial compounds, such as azithromycin, clarithromycin, dirithromycin, erythromycin and troleandomycin; lincomycin antibacterial compounds, such as lincomycin, clindamycin and pirlimycin; ketolide antibacterial compounds, such as telithromycin; newquinolone antibacterial compounds, such as ciprofloxacin, ofloxacin and sitafloxacin; glycopeptide antibacterial compounds, such as vancomycin and teicoplanin; streptogramin antibacterial compounds, such as quinupristin and dalfopristin; tetracycline antibacterial compounds, such as demeclocycline, doxycycline, minocycline, oxytetracycline and tetracycline; chloramphenicol antibacterial compounds, such as chloramphenicol; quinolone antibacterial compounds, such as ciprofloxacin, enoxacin, gatifloxacin, levofloxacin, lomefloxacin, moxifloxacin, norfloxacin, ofloxacin and trovafloxacin; peptide antibacterial compounds, such as bacitracin, colistin and polymyxin B; aminoglycoside antibacterial compounds, such as amikacin, gentamicin, kanamycin, capreomycin, neomycin, netilmicin, streptomycin and tobramycin; monobactam antibacterial compounds, such as aztreonam; nitroimidazole antibacterial compounds, such as metronidazole; and fosfomycin antibacterial compounds, such as fosfomycin.

The agent having antibacterial activity against normal flora bacteria in the oral cavity may be a single type of agent, or may be a combination of two, three, four, five, six, seven, eight, nine, or ten types or more of agents.

The active ingredient of the composition of the present invention may be a phage exhibiting bacteriolytic activity against gut bacteria that are increased in a patient with pulmonary hypertension compared to those in a healthy subject. Genetic engineering techniques for the generation of a phage exhibiting bacteriolytic activity against specific bacteria or techniques for isolation of such a phage from the environment or culture of the phage have already been established (Trends Biotechnol. 2010 Dec;28(12):591-595. doi: 10.1016/j.tibtech.2010.08.001. Epub 2010 Aug 31; Bacteriophage. 2011 Mar-Apr; 1(2): 111-114. doi: 10.4161/bact.1.2.14590). A phage exhibiting bacteriolytic activity against gut bacteria that are increased in a patient with pulmonary hypertension compared to those in a healthy subject is useful as an active ingredient of the composition of the present invention.

The active ingredient of the composition of the present invention may be a substance capable of increasing gut bacteria that are decreased in a patient with pulmonary hypertension compared to those in a healthy subject. The composition comprising a substance capable of increasing gut bacteria that are decreased in a patient with pulmonary hypertension compared to those in a healthy subject may be a composition comprising gut bacteria that are decreased in a patient with pulmonary hypertension compared to those in a healthy subject (hereinafter referred to as the composition comprising the gut bacteria). The gut bacteria that are decreased in a patient with pulmonary hypertension compared to those in a healthy subject can be isolated from human feces according to a conventional method. The gut bacteria that are decreased in a patient with pulmonary hypertension compared to those in a healthy subject may be a bacterial stock prepared from isolated bacteria. The gut bacteria contained in the composition comprising the gut bacteria are any one or more types of gut bacteria that are decreased in a patient with pulmonary hypertension compared to those in a healthy subject, and may be a combination of 2, 3, 4, 5, 6, 7, 8, 9, 10, 11, 12, 13, 14, 15, 16, 17, 18, 19 or 20 types of such bacteria. When the composition comprises two or more types of the bacteria, the percentage of each type of bacteria in the composition is not particularly limited and may be determined as appropriate depending on the age, pathological conditions, severity, etc. of the patient with pulmonary hypertension.

The composition comprising the gut bacteria may further contain a culture medium in which the bacteria are grown. The composition comprising gut bacteria that are decreased in a patient with pulmonary hypertension compared to those in a healthy subject may further contain a fraction of culture medium containing a secretory or metabolic product produced from the bacteria.

The bacteria contained in the composition comprising the gut bacteria may be live bacteria, attenuated or inactivated bacteria, or killed bacteria, e.g., heat-killed bacteria.

The composition comprising the gut bacteria may be provided in the form of a pharmaceutical composition. When provided in the form of a pharmaceutical composition, the composition comprising the gut bacteria may be formulated by pharmaceutical formulation techniques known in the art. The composition comprising the gut bacteria may be orally administered in the form of, for example, a capsule, a tablet, a pill, a sachet formulation, a solution, a powder, granules, fine granules, a film-coated formulation, a pellet, a troche, a sublingual formulation, a chewable formulation, a buccal formulation, a paste, a syrup, a suspension, an elixir or an emulsion. The composition may also be administered in the form of a suppository or an enema.

The composition comprising the gut bacteria may be provided in the form of a food composition. The food composition may be a food product, including dietary supplements, health foods, functional foods, foods for special health use, foods for sick people, etc. The form of the food composition may be any one of, for example, but is not limited to, drinks such as tea drinks, refreshing drinks, carbonated drinks, nutritional drinks, fruit juice, and lactic drinks; noodles such as buckwheat noodles, wheat noodles, Chinese noodles, and instant noodles; sweets and bakery products, such as drops, candies, chewing gum, chocolate, snacks, biscuits, jellies, jam, cream, pastries, and bread; fishery and livestock products, such as fish sausages, ham, and sausages; dairy products such as processed milk and fermented milk; fats, oils and processed foods thereof, such as vegetable oil, oil for deep frying, margarine, mayonnaise, shortening, whipped cream, and dressing; seasonings such as sauce and dipping sauce; retort pouch foods such as curry, stew, rice-bowl cuisine, porridge, and rice soup; and frozen desserts such as ice cream, sherbet, and shaved ice.

A pharmacologically acceptable carrier or a carrier acceptable for ingestion as a food or a drink may be used to formulate the composition comprising the gut bacteria. Examples of such a carrier include sterilized water, physiological saline, vegetable oils, solvents, bases, emulsifiers, suspending agents, surfactants, stabilizers, flavoring agents, fragrances, excipients, antiseptics, binders, diluents, isotonic agents, soothing agents, fillers, disintegrants, buffering agents, coating agents, lubricants, colorants, sweeteners, thickening agents, flavor improvers, and solubilizing agents.

The composition comprising the gut bacteria may further contain an additional ingredient capable of efficiently delivering the bacteria to the large intestine when the composition is orally ingested. Such an additional ingredient may be, for example, a pH sensitive composition. Specifically, the additional ingredient may be a buffered sachet formulation or an enteric polymer that is capable of releasing its content when the pH becomes alkaline after passing through the stomach. When the composition comprising the gut bacteria is formulated using a pH sensitive composition, the pH threshold at which the pH sensitive composition is decomposed is preferably within the alkaline pH range at the distal end of the stomach, where the intraluminal pH changes from acid to alkaline. Specifically, the pH threshold at which the pH sensitive composition is decomposed is preferably about 6.8 to about 7.5.

The additional ingredient capable of efficiently delivering the bacteria to the large intestine when the composition comprising the gut bacteria is orally ingested may be a material capable of delaying the release of the content (e.g., the composition comprising the gut bacteria of the present invention) for about 3 to 5 hours, which correspond to the small bowel transit time, thereby ensuring the delivery of the content to the large intestine. The composition containing such a material may be, for example, a pharmaceutical formulation containing a hydrogel as a core material. The hydrogel is hydrated and swells upon contact with gastrointestinal fluid to let the content elute efficiently, in particular, to release the content primarily to the large intestine.

The additional ingredient capable of efficiently delivering the bacteria to the large intestine when the composition comprising the gut bacteria is orally ingested may be a selective coating material, such as a biodegradable polymer, a gradually hydrolyzable polymer, a gradually water-soluble polymer, and an enzymatically degradable polymer. A coating material capable of efficiently delaying the release of the content may be, for example, a cellulose-based polymer such as hydroxypropyl cellulose, an acrylic acid polymer or copolymer such as a methacrylate polymer, or a vinyl polymer or copolymer such as polyvinylpyrrolidone.

A composition capable of delivering the content to the large intestine may be, for example, a bioadhesive composition that specifically adheres to the mucosa of the large intestine (e.g., the polymer described in U.S. Pat. No. 6,368,586), or a composition containing a protease inhibitor, especially for protecting a biologic from degradation by protease activity in the digestive tract.

The composition comprising the gut bacteria of the present invention may be used in combination with a prebiotic substance that selectively acts on the bacteria in the composition so that the growth of the bacteria is promoted as compared to that of other symbiotic bacteria in humans. Such a prebiotic substance may be, for example, an indigestible oligosaccharide, an indigestible starch, or a dietary fiber.

The bacteria serving as an active ingredient of the composition comprising the gut bacteria can be produced by fermentation techniques. For example, the bacterial active ingredient may be produced in an anaerobic fermentation tank that supports rapid growth of the bacteria. The anaerobic fermentation tank may be, for example, a stirred tank reactor or a disposable WAVE Bioreactor. The bacterial active ingredient may be grown on BL agar medium or in other similar types of culture media free of animal components. The bacterial active ingredient can be purified and/or concentrated from a fermentation broth by known techniques, such as centrifugation or filtration. The bacterial active ingredient may be dried or lyophilized.

The dosage or intake of the composition comprising the gut bacteria can be empirically determined considering various factors including the age, body weight, sex, symptoms and health conditions of a patient with pulmonary hypertension and the type of the composition (a pharmaceutical, a food or a drink). For example, the dosage or intake for a single administration may typically be 0.01 mg/kg body weight to 100 mg/kg body weight, or 1 mg/kg body weight to 10 mg/kg body weight.

The composition for improving pulmonary hypertension comprising, as an active ingredient, a substance capable of increasing gut bacteria that are decreased in a patient with pulmonary hypertension compared to those in a healthy subject may be used in combination with a composition for improving pulmonary hypertension comprising a substance capable of reducing gut bacteria that are increased in a patient with pulmonary hypertension compared to those in a healthy subject as an active ingredient, and/or with a composition for improving pulmonary hypertension comprising a substance capable of inhibiting a specific metabolic pathway (described later) as an active ingredient, and/or with a composition for improving pulmonary hypertension comprising a short-chain fatty acid or a salt thereof (described later) as an active ingredient. The term "used in combination" as used herein means that the administration periods of two or more compositions or agents overlap, but the two or more compositions or agents are not required to be simultaneously administered or ingested.

The inventors analyzed the functional metabolic profile of gut microbiota using data from gut microbiota in patients with pulmonary hypertension and in healthy subjects and found that the functions relating to some metabolic pathways are enhanced in the patients with pulmonary hypertension. The metabolic pathways that are enhanced in patients with pulmonary hypertension include the mevalonate pathway, the mannosylglycerate synthesis pathway, the methylglyoxal degradation pathway, the D-glucarate degradation pathway, the TCA cycle VIII pathway, the heme biosynthesis pathway, the nitrate metabolism pathway, etc. Therefore, the composition of the present invention may comprise, as an active ingredient, a substance capable of inhibiting at least one pathway selected from the group consisting of the mevalonate pathway, the mannosylglycerate synthesis pathway, the methylglyoxal degradation pathway, the D-glucarate degradation pathway, the TCA cycle VIII pathway, the heme biosynthesis pathway and the nitrate metabolism pathway.

The active ingredient of the composition of the present invention may be an HMG-CoA reductase inhibitor. The HMG-CoA reductase inhibitor is a substance known to inhibit the mevalonate pathway. The HMG-CoA reductase inhibitor may include, for example, but is not limited to, mevastatin (compactin) (see USP3983140), pravastatin (see JP S57-2240 A (USP4346227)), lovastatin (see JP S57-163374 A (USP4231938)), simvastatin (see JP S56-122375 A (USP4444784)), fluvastatin (see JP S60-500015 A (USP4739073)), atorvastatin (see JP H03-58967 A (USP5273995)), rosuvastatin (see JP H05-178841 A (USP5260440)), and pitavastatin (see JP H01-279866 A (USP5854259 and USP5856336)).

The inventors also found that the short-chain fatty acid levels in the feces of patients with pulmonary hypertension are significantly reduced compared to those in healthy subjects. The inventors further found that oral administration of a short-chain fatty acid to a rat model of pulmonary hypertension induced by exposure to hypoxia results in improvement of the phenotypes of pulmonary hypertension as compared to that in a vehicle administration group. These findings indicate that a short-chain fatty acid may be capable of changing the intestinal environment of patients with pulmonary hypertension to be closer to that of healthy subjects. In other words, a short-chain fatty acid may be capable of changing the composition of gut microbiota in patients with pulmonary hypertension to be closer to that of healthy subjects. Therefore, the active ingredient of the composition of the present invention may be a short-chain fatty acid or a salt thereof. In other words, the present invention provides a composition for improving pulmonary hypertension, comprising a short-chain fatty acid or a salt thereof as an active ingredient (hereinafter referred to as the composition comprising a short-chain fatty acid).

The short-chain fatty acid may be a fatty acid of six carbon atoms or less and specific examples of the short-chain fatty acid include acetic acid, propionic acid, butyric acid, isobutyric acid, valeric acid, isovaleric acid, caproic acid, lactic acid, and succinic acid. The short-chain fatty acid is preferably butyric acid, isobutyric acid, valeric acid, isovaleric acid, propionic acid, acetic acid, succinic acid or lactic acid, and is more preferably butyric acid.

The fatty acid may be in the form of a free base or a salt. The fatty acid in the form of a salt will have increased water solubility and increased physiological efficacy. A salt of the fatty acid may be any pharmaceutically acceptable salt. Examples of a salt of the fatty acid include salts with an alkali metal such as lithium, sodium and potassium; salts with an alkaline earth metal such as magnesium and calcium; salts with an inorganic base such as ammonium hydroxide; salts with a basic amino acid such as arginine, lysine, histidine and ornithine; and salts with an organic base such as monoethanolamine, diethanolamine and triethanolamine. A salt of the fatty acid is preferably an alkali metal salt or an alkaline earth metal salt.

The composition comprising a short-chain fatty acid may be provided in the form of a pharmaceutical composition. When provided in the form of a pharmaceutical composition, the composition comprising a short-chain fatty acid may be formulated by pharmaceutical formulation techniques known in the art. The composition comprising a short-chain fatty acid may be orally administered in the form of, for example, a capsule, a tablet, a pill, a sachet formulation, a solution, a powder, granules, fine granules, a film-coated formulation, a pellet, a troche, a sublingual formulation, a chewable formulation, a buccal formulation, a paste, a syrup, a suspension, an elixir or an emulsion. The composition may also be administered in the form of a suppository or an enema.

The composition comprising a short-chain fatty acid may be provided in the form of a food composition. The food composition may be a food product, including dietary supplements, health foods, functional foods, foods for special health use, foods for sick people, etc. The form of the food composition may be any one of, for example, but is not limited to, drinks such as tea drinks, refreshing drinks, carbonated drinks, nutritional drinks, fruit juice, and lactic drinks; noodles such as buckwheat noodles, wheat noodles, Chinese noodles, and instant noodles; sweets and bakery products, such as drops, candies, chewing gum, chocolate, snacks, biscuits, jellies, jam, cream, pastries, and bread; fishery and livestock products, such as fish sausages, ham, and sausages; dairy products such as processed milk and fermented milk; fats, oils and processed foods thereof, such as vegetable oil, oil for deep frying, margarine, mayonnaise, shortening, whipped cream, and dressing; seasonings such as sauce and dipping sauce; retort pouch foods such as curry, stew, rice-bowl cuisine, porridge, and rice soup; and frozen desserts such as ice cream, sherbet, and shaved ice.

A pharmacologically acceptable carrier or a carrier acceptable for ingestion as a food or a drink may be used to formulate the composition comprising a short-chain fatty acid. Examples of such a carrier include sterilized water, physiological saline, vegetable oils, solvents, bases, emulsifiers, suspending agents, surfactants, stabilizers, flavoring agents, fragrances, excipients, antiseptics, binders, diluents, isotonic agents, soothing agents, fillers, disintegrants, buffering agents, coating agents, lubricants, colorants, sweeteners, thickening agents, flavor improvers, and solubilizing agents.

The dosage or intake of the composition comprising a short-chain fatty acid can be empirically determined considering various factors including the age, body weight, sex, symptoms and health conditions of a patient with pulmonary hypertension and the type of the composition (a pharmaceutical, a food or a drink). For example, the dosage or intake for a single administration may typically be 0.01 mg/kg body weight to 100 mg/kg body weight, or 1 mg/kg body weight to 10 mg/kg body weight.

### Method for predicting the prognosis of patients with pulmonary hypertension

The present invention provides a method for predicting the prognosis of a patient with pulmonary hypertension (hereinafter referred to as the method for the prognosis prediction of the present invention). The method for the prognosis prediction of the present invention comprises detecting one or more types of bacteria selected from bacteria belonging to the family Micrococcaceae, Streptococcaceae, Pasteurellaceae, Veillonellaceae or Lactobacillaceae. In the method for the prognosis prediction of the present invention, a poor prognosis refers to a high probability of registration for lung transplantation in the future, a high risk of developing right-sided heart failure in the future, and/or a high risk of death in relation to pulmonary hypertension in the future.

Specifically, the method for the prognosis prediction of the present invention preferably comprises
(1) detecting one or more types of bacteria selected from bacteria belonging to the family Micrococcaceae, Streptococcaceae, Pasteurellaceae, Veillonellaceae or Lactobacillaceae in DNA extracted from feces of a patient with pulmonary hypertension to be subjected to the prediction of prognosis, and
(2) determining that the patient with pulmonary hypertension has a poor prognosis when the one or more types of bacteria are detected.

The Micrococcaceae bacteria may be the bacteria belonging to the genus *Rothia,* and may be *Rothia mucilaginosa, Rothia dentocariosa,* or *Rothia aeria.* The Streptococcaceae bacteria may be the bacteria belonging to the genus *Streptococcus,* and may be *Streptococcus salivarius, Streptococcus parasanguinis, Streptococcus vestibularis, Streptococcus oligofermentans*, *Streptococcus anginosus, Streptococcus mitis, Streptococcus oralis, Streptococcus pneumoniae, Streptococcus mutans, Streptococcus intermedius, Streptococcus peroris,* or *Streptococcus tigurinus.* The Pasteurellaceae bacteria may be the bacteria belonging to the genus *Haemophilus,* and may be *Haemophilus parainfluenzae.* The Veillonellaceae bacteria may be the bacteria belonging to the genus *Veillonella,* and may be *Veillonella parvula, Veillonella atypica*, *Veillonella disper, Veillonella denticariosi,* or *Veillonella rogosae.* The Lactobacillaceae bacteria may be the bacteria belonging to the genus *Lactobacillus,* and may be *Lactobacillus delbrueckii, Lactobacillus acidophilus, Lactobacillus crispatus, Lactobacillus gasseri, Lactobacillus helveticus, Lactobacillus johnsonii, Lactobacillus kefiranofaciens, Lactobacillus paragasseri, Lactobacillus casei, Lactobacillus paracasei, Lactobacillus rhamnosus, Lactobacillus sakei, Lactobacillus curvatus, Lactobacillus salivarius, Lactobacillus plantarum, Lactobacillus pentosus, Lactobacillus fermentum, Lactobacillus reuteri, Lactobacillus brevis, Lactobacillus fructivorans, Lactobacillus lindneri, Lactobacillus sanfranciscensis,* or *Lactobacillus kunkeei.*

Extraction of DNA from feces can be performed by a known DNA extraction method. Detection of the bacteria can be performed by, for example, metagenomic analysis, PCR, ELISA, stool culture tests, antigen tests, etc. Metagenomic analysis may be 16S rRNA metagenomic analysis or whole metagenomic analysis. Metagenomic analysis can be performed by a known method. PCR can be performed by a known method using DNA extracted from feces as a template. The primers used in PCR may be, but are not limited to, the following primers.
- 16S primer sets that can detect all bacteria.
   16S (Total Bacteria)_F: GTGSTGCAYGGYTGTCGTCA (SEQ ID NO: 1)
   16S (Total Bacteria)_R: ACGTCRTCCMCACCTTCCTC (SEQ ID NO: 2)
- Primer sets that can detect Micrococcaceae bacteria.
   RM_F: GCCTAGCTTGCTAGGTGGAT (SEQ ID NO: 3)
   RM_R: GCAGGTACCGTCAATCTCTC (SEQ ID NO: 4)
- Primer sets that can detect Streptococcaceae bacteria.
   Str1_F: GTACAGTTGCTTCAGGACGTATC (SEQ ID NO: 5)
   Str2_R: ACGTTCGATTTCATCACGTTG (SEQ ID NO: 6)
- Primer sets that can detect Pasteurellaceae bacteria.
   p84_F: GACGGAAAGACCCCGTGAACCT (SEQ ID NO: 7)
   p85_R: GGCAAGTTTCGTGCTTAGAT (SEQ ID NO: 8)

The nucleotide sequence of the genome of the bacteria of interest can be acquired from a known database, and an appropriate primer set can be designed based on the nucleotide sequence and used for the detection of the bacteria at the genus or species level.

A patient with pulmonary hypertension to be subjected to the prediction of prognosis can be determined to have a poor prognosis when any one type of bacteria belonging to the family Micrococcaceae, Streptococcaceae, Pasteurellaceae, Veillonellaceae or Lactobacillaceae are detected. A patient with pulmonary hypertension to be subjected to the prediction of prognosis can be determined to have a poor prognosis when Streptococcaceae bacteria are detected with an abundance of 1% or more. A patient with pulmonary hypertension to be subjected to the prediction of prognosis can be determined to have a poor prognosis when Pasteurellaceae bacteria are detected with an abundance of 0.1% or more.

### Method for assisting the determination of severity of patients with pulmonary hypertension

The present invention provides a method for assisting the determination of the severity of a patient with pulmonary hypertension (hereinafter referred to as the method for assisting the determination of the severity of the present invention). The method for assisting the determination of the severity of the present invention comprises detecting one or more types of bacteria selected from bacteria belonging to the family Micrococcaceae, Streptococcaceae, Pasteurellaceae, Veillonellaceae or Lactobacillaceae. The severity of a patient with pulmonary hypertension can be determined based on, for example, the WHO Functional Classification of Pulmonary Hypertension (Table 2).

**Table 2: WHO Functional Classification of Pulmonary Hypertension (PH)**

| | |
|---|---|
| Class I | Patients with PH where there is no limitation of physical activity. Normal physical activity does not cause dyspnea, fatigue, chest pain, or presyncope. |
| Class II | Patients with PH who have mild limitation of physical activity. There is no discomfort at rest, but normal physical activity causes dyspnea, fatigue, chest pain, or presyncope. |
| Class III | Patients with PH who have marked limitation of physical activity. There is no discomfort at rest, but less than normal physical activity causes dyspnea, fatigue, chest pain, or presyncope. |
| Class IV | Patients with PH who are unable to perform any physical activity without symptoms and who may manifest signs of right ventricular failure. Dyspnea and/or fatigue may be present at rest, and symptoms are increased by almost any physical activity. |

The method for assisting the determination of the severity of the present invention preferably comprises
(1) detecting one or more types of bacteria selected from bacteria belonging to the family Micrococcaceae, Streptococcaceae, Pasteurellaceae, Veillonellaceae or Lactobacillaceae in DNA extracted from feces of a patient with pulmonary hypertension to be subjected to the determination of the severity of pulmonary hypertension, and
(2) assisting the determination that the patient has severe pulmonary hypertension when Micrococcaceae bacteria are detected, when Streptococcaceae bacteria are detected with an abundance of 0.5% or more, and/or when Pasteurellaceae bacteria are detected with an abundance of 0.1% or more.

The Micrococcaceae bacteria may be the bacteria belonging to the genus *Rothia,* and may be *Rothia mucilaginosa, Rothia dentocariosa,* or *Rothia aeria.* The Streptococcaceae bacteria may be the bacteria belonging to the genus *Streptococcus,* and may be *Streptococcus salivarius, Streptococcus parasanguinis, Streptococcus vestibularis, Streptococcus oligofermentans, Streptococcus anginosus, Streptococcus mitis, Streptococcus oralis, Streptococcus pneumoniae, Streptococcus mutans, Streptococcus intermedius, Streptococcus peroris,* or *Streptococcus tigurinus.* The Pasteurellaceae bacteria may be the bacteria belonging to the genus *Haemophilus,* and may be *Haemophilus parainfluenzae.* The Veillonellaceae bacteria may be the bacteria belonging to the genus *Veillonella,* and may be *Veillonella parvula, Veillonella atypica, Veillonella disper, Veillonella denticariosi,* or *Veillonella rogosae.* The Lactobacillaceae bacteria may be the bacteria belonging to the genus *Lactobacillus,* and may be *Lactobacillus delbrueckii, Lactobacillus acidophilus, Lactobacillus crispatus, Lactobacillus gasseri, Lactobacillus helveticus, Lactobacillus johnsonii, Lactobacillus kefiranofaciens, Lactobacillus paragasseri, Lactobacillus casei, Lactobacillus paracasei, Lactobacillus rhamnosus, Lactobacillus sakei, Lactobacillus curvatus, Lactobacillus salivarius, Lactobacillus plantarum, Lactobacillus pentosus, Lactobacillus fermentum, Lactobacillus reuteri, Lactobacillus brevis, Lactobacillus fructivorans, Lactobacillus lindneri, Lactobacillus sanfranciscensis,* or *Lactobacillus kunkeei.*

The method for assisting the determination of the severity of the present invention may comprise detecting one or more types of bacteria selected from bacteria belonging to the genus *Streptococcus, Rothia* or *Veillonella.* In this case, the method preferably comprises
(I) detecting one or more types of bacteria selected from bacteria belonging to the genus *Streptococcus, Rothia* or *Veillonella* in DNA extracted from feces of a patient with pulmonary hypertension to be subjected to the determination of the severity of pulmonary hypertension, and
(II) assisting the determination that the patient has severe pulmonary hypertension when *Streptococcus* bacteria are detected with an abundance of 10.8% or more, when *Rothia* bacteria are detected with an abundance of 0.33% or more, and/or when *Veillonella* bacteria are detected with an abundance of 4.56% or more.

Extraction of DNA from feces can be performed by a known DNA extraction method. Detection of the bacteria can be performed by, for example, metagenomic analysis, PCR, ELISA, stool culture tests, antigen tests, etc. Metagenomic analysis may be 16S rRNA metagenomic analysis or whole metagenomic analysis. Metagenomic analysis can be performed by a known method. PCR can be performed by a known method using DNA extracted from feces as a template. The primer sets described in the method for the prognosis prediction of the present invention as above can be suitably used as the primers for PCR.

When any one or more types of Micrococcaceae bacteria, Streptococcaceae bacteria and Pasteurellaceae bacteria are detected at an abundance of more than the percentage described in step (2), the results can be used to assist the determination that the patient has severe pulmonary hypertension. Alternatively, when any one or more types of *Streptococcus* bacteria, *Rothia* bacteria and *Veillonella* bacteria are detected at an abundance of more than the percentage described in step (II), the results can be used to assist the determination that the patient has severe pulmonary hypertension.

### Method for assisting the diagnosis of pulmonary hypertension

The present invention provides a method for assisting the diagnosis of pulmonary hypertension (hereinafter referred to as the method for assisting the diagnosis of pulmonary hypertension of the present invention). The method for assisting the diagnosis of pulmonary hypertension of the present invention comprises measuring an IgA level in the feces of a subject, and comparing the measured IgA level with that of a healthy subject.

The method for assisting the diagnosis of pulmonary hypertension of the present invention preferably comprises
(1) measuring an IgA level in the feces of a subject, and
(2) assisting the diagnosis that the subject is suffering from pulmonary hypertension when the IgA level in the feces of the subject is higher than that of a healthy subject.

The IgA level in feces can be determined by suspending the feces in an appropriate solution such as PBS to prepare a diluted suspension, and measuring the IgA level in the suspension using a commercially available IgA measurement kit etc. The IgA level in the feces of a healthy subject as a control may be measured simultaneously with the measurement of the IgA level in the feces of the subject, or may be a previously accumulated value. When the IgA level in the feces of a subject is higher than that of a healthy subject, the results can be used to assist the diagnosis that the subject has developed pulmonary hypertension. In particular, when the IgA level in the feces of a subject is 120% or more, 130% or more, 140% or more, 150% or more, 170% or more, 180% or more, 190% or more, or 200% or more as compared to that of a healthy subject assumed as 100%, the results can be used to assist the diagnosis that the subject has developed pulmonary hypertension.

### Method for assisting the diagnosis of portal hypertension-associated pulmonary hypertension

The present invention provides a method for assisting the diagnosis of portal hypertension-associated pulmonary hypertension (hereinafter referred to as the method for assisting the diagnosis of portal hypertension-associated pulmonary hypertension of the present invention). The method for assisting the diagnosis of portal hypertension-associated pulmonary hypertension of the present invention comprises detecting one or more types of bacteria selected from *Bifidobacterium* bacteria, *Dorea* bacteria, and *Blautia* bacteria in gut microbiota in a subject.

The method for assisting the diagnosis of portal hypertension-associated pulmonary hypertension of the present invention preferably comprises
(1) detecting one or more types of bacteria selected from bacteria belonging to the genus *Bifidobacterium, Dorea* or *Blautia* in DNA extracted from feces of a subject, and
(2) assisting the diagnosis that the subject is suffering from portal hypertension-associated pulmonary hypertension when the one or more types of bacteria are detected.

Extraction of DNA from feces can be performed by a known DNA extraction method. Detection of the bacteria can be performed by, for example, metagenomic analysis, PCR, ELISA, stool culture tests, antigen tests, etc. Metagenomic analysis may be 16S rRNA metagenomic analysis or whole metagenomic analysis. Metagenomic analysis can be performed by a known method. PCR can be performed by a known method using DNA extracted from feces as a template.

### Method for assisting the diagnosis of chronic thromboembolic pulmonary hypertension

The present invention provides a method for assisting the diagnosis of chronic thromboembolic pulmonary hypertension (hereinafter referred to as the method for assisting the diagnosis of chronic thromboembolic pulmonary hypertension of the present invention). The method for assisting the diagnosis of chronic thromboembolic pulmonary hypertension of the present invention comprises detecting *Klebsiella* bacteria in gut microbiota in a subject.

The method for assisting the diagnosis of chronic thromboembolic pulmonary hypertension of the present invention preferably comprises
(1) detecting *Klebsiella* bacteria in DNA extracted from feces of a subject, and
(2) assisting the diagnosis that the subject is suffering from chronic thromboembolic pulmonary hypertension when *Klebsiella* bacteria are detected.

Extraction of DNA from feces can be performed by a known DNA extraction method. Detection of the bacteria can be performed by, for example, metagenomic analysis, PCR, ELISA, stool culture tests, antigen tests, etc. Metagenomic analysis may be 16S rRNA metagenomic analysis or whole metagenomic analysis. Metagenomic analysis can be performed by a known method. PCR can be performed by a known method using DNA extracted from feces as a template.

The present invention further includes the following.
(a1) A method for improving pulmonary hypertension, comprising administering an effective amount of an agent having antibacterial activity against normal flora bacteria in the oral cavity of a patient with pulmonary hypertension to normalize gut microbiota in the patient.
(a2) A method for improving pulmonary hypertension, comprising administering an effective amount of a phage exhibiting bacteriolytic activity against gut bacteria that are increased in a patient with pulmonary hypertension as compared to those in a healthy subject to normalize gut microbiota in the patient.
(a3) A method for improving pulmonary hypertension, comprising administering an effective amount of at least one type of gut bacteria that are increased in a patient with pulmonary hypertension compared to those in a healthy subject to normalize gut microbiota in the patient.
(a4) A method for improving pulmonary hypertension, comprising administering an effective amount of an HMG-CoA reductase inhibitor to a patient with pulmonary hypertension.
(a5) A method for improving pulmonary hypertension, comprising administering an effective amount of a short-chain fatty acid or a salt thereof to a patient with pulmonary hypertension.
(b1) An agent having antibacterial activity against normal flora bacteria in an oral cavity for use in the improvement of pulmonary hypertension by normalizing gut microbiota in a patient with pulmonary hypertension.
(b2) A phage exhibiting bacteriolytic activity against gut bacteria that are increased in a patient with pulmonary hypertension compared to those in a healthy subject for use in the improvement of pulmonary hypertension by normalizing gut microbiota in a patient with pulmonary hypertension.
(b3) At least one type of gut bacteria that are increased in a patient with pulmonary hypertension compared to those in a healthy subject for use in the improvement of pulmonary hypertension by normalizing gut microbiota in a patient with pulmonary hypertension.
(b4) An HMG-CoA reductase inhibitor for use in the improvement of pulmonary hypertension.
(b5) A short-chain fatty acid or a salt thereof for use in the improvement of pulmonary hypertension.
(c1) Use of an agent having antibacterial activity against normal flora bacteria in an oral cavity for the production of a medicament for improving pulmonary hypertension by normalizing gut microbiota in a patient with pulmonary hypertension.
(c2) Use of a phage exhibiting bacteriolytic activity against gut bacteria that are increased in a patient with pulmonary hypertension compared to those in a healthy subject for the production of a medicament for improving pulmonary hypertension by normalizing gut microbiota in a patient with pulmonary hypertension.
(c3) Use of at least one type of gut bacteria that are increased in a patient with pulmonary hypertension compared to those in a healthy subject for the production of a medicament or a food composition for improving pulmonary hypertension by normalizing gut microbiota in a patient with pulmonary hypertension.
(c4) Use of an HMG-CoA reductase inhibitor in the production of a medicament for improving pulmonary hypertension.
(c5) Use of a short-chain fatty acid or a salt thereof in the production of a medicament for improving pulmonary hypertension.

### EXAMPLES

The present invention will be described in more detail below with reference to Examples, but the present invention is not limited thereto.

### Example 1: Examination of gut microbiota in animal models of pulmonary hypertension

### A. Experimental materials and methods

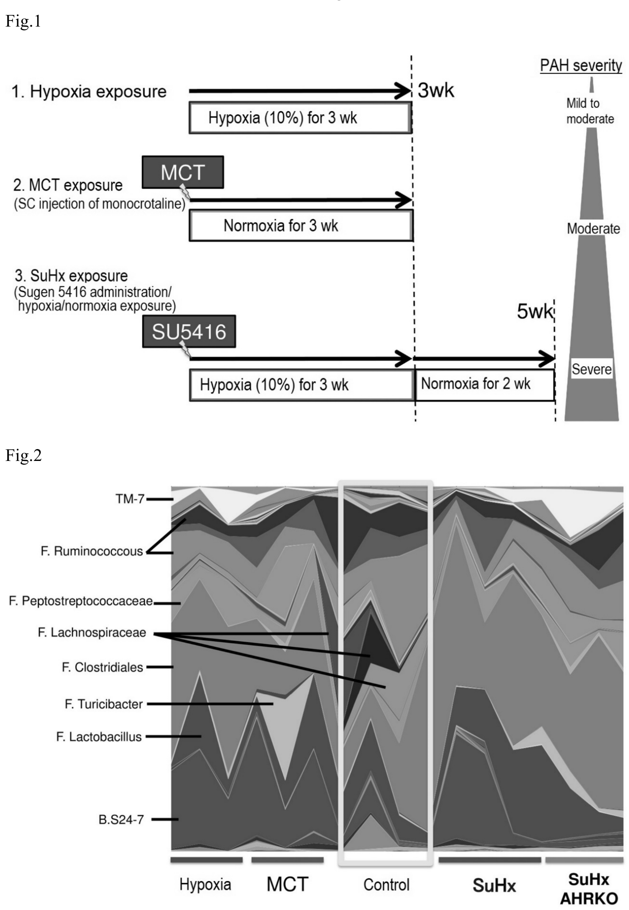

### (1) Preparation of animal models of pulmonary hypertension (Fig. 1)

### (1-1) Preparation of rat model of pulmonary hypertension induced by exposure to hypoxia

Six-week-old male SD rats (purchased from Oriental Yeast) were used. The rats were housed continuously in a hypoxic chamber at 10% oxygen for three weeks to prepare a rat model of pulmonary hypertension induced by exposure to hypoxia (Hx).

### (1-2) Preparation of rat model of pulmonary hypertension induced by administration of monocrotaline

Six-week-old male SD rats (purchased from Oriental Yeast) were used. The rats received subcutaneous injection of Crotaline (Sigma) at 60 mg/kg and were housed under standard normoxic conditions (normoxia) for three weeks to prepare a rat model of pulmonary hypertension induced by administration of monocrotaline (MCT).

### (1-3) Preparation of rat model of pulmonary hypertension induced by Sugen 5416/hypoxia/normoxia

Six-week-old male SD rats (purchased from Oriental Yeast) were used. The rats received subcutaneous administration of a VEGFR2 inhibitor Sugen 5416 (MedChemExpress) at 20 mg/kg and were housed continuously in a hypoxic chamber at 10% oxygen for three weeks. The rats were then transferred to and housed under standard normoxic conditions (normoxia) for two weeks to prepare a rat model of pulmonary hypertension induced by Sugen 5416/hypoxia/normoxia (SuHx).

### (1-4) Control animals

Untreated six-week-old male SD rats (purchased from Oriental Yeast) were used as a control group. As another control animal group, rats deficient in the aryl hydrocarbon receptor (AHR) (AHRKO rats) were subjected to Sugen 5416/hypoxia/normoxia. The AHRKO rats were generated at the Institute of Experimental Animal Sciences of Osaka University Medical School. Specifically, a guide RNA directed to the target site adjacent to the PAM sequence in Exon-2 of the AHR gene was designed and used to gene editing in SD rats using the CRISPR-Cas9 system.

### (2) Measurement of right ventricular systolic pressure

Rats were anesthetized by inhalation of isoflurane (Pfizer) for induction of sedation and analgesia. Throughout the procedure, the body temperature of the rats was maintained at 37 to 38°C using a rectal thermistor coupled with a thermostatically controlled heating pad. The trachea was cannulated, and the lungs were ventilated with a mouse ventilator (Harvard Apparatus) at a tidal volume of 10 µL/g at a frequency of 70/min. A polyethylene tube was inserted into the right external jugular vein and advanced into the right ventricle to measure right ventricular pressure (RVP). RVP signals were detected by a pressure transducer (MLT0670; ADInstruments), and the signals were relayed to pressure amplifiers (ML117; ADInstruments), and then were sampled continuously with a PowerLab system (ADInstruments, Colorado Springs, CO) and recorded on a computer using Chart software (ADInstruments). Heart rate was derived from the right ventricular systolic peaks.

### (3) Ratio of right ventricle to left ventricle weight

After right ventricular systolic pressure measurement, the rats were killed by anesthesia overdose, and the heart was excised. The atria were removed, and the right ventricle (RV) was separated from the left ventricle (LV) and septum. The tissues were blotted, and the weight of the right ventricle (RV) and the weight of the left ventricle (LV) + septum were measured. The ratio of RV to LV + septum weight (the ratio of RV to LV weight) was calculated and used as an index of right ventricular hypertrophy.

### (4) Metagenomic analysis of 16S rRNA of gut microbiota

DNA was extracted from the fecal samples using NucleoSpin DNA Stool (Macherey-Nagel). Libraries were prepared according to the protocol of 16S Metagenomic Sequencing Library Preparation (Illumina) with a primer set (27Fmod: 5'-AGRGTTTGATCMTGGCTCAG-3' (SEQ ID NO: 9) and 338R: 5'-TGCTGCCTCCCGTAGGAGT-3' (SEQ ID NO: 10)) targeting the V1-V2 regions of the 16S ribosomal RNA. Amplicons were subjected to 251 bp paired-end sequencing on the MiSeq system (Illumina). The paired-end reads were merged using PEAR (sco.hits.org/exelixis/web/software/pear/) and trimmed using BBtrim (bbmap.sourceforge.net). Twenty thousand reads per sample were randomly selected using random_sequence_sample.pl (ualberta.ca/~stothard/software.html). The processed sequences were clustered into OTUs (operational taxonomic units) defined by a 97% similarity cut-off using UCLUST version 1.2.33q. Representative sequences for each OTU were then classified taxonomically by using RDP Classifier version 2.2 and the Greengenes 13_8 database. Bioinformatic analysis of the bacterial flora was performed using QIIME version 1.9.1.

### (5) Evaluation of pathological changes in pulmonary hypertension by administration of antibiotic cocktail

### (5-1) Administration of antibiotic cocktail to Hx

Six-week-old male SD rats were housed in a hypoxic chamber at 10% oxygen for three weeks with access to autoclaved drinking water containing four antibiotics (1 g/L ampicillin, 1 g/L fradiomycin, 1 g/L metronidazole and 0.5 g/L vancomycin) during the feeding period (Hx antibiotic group). As controls, two groups were constituted: the first is a Hx group in which the rats were housed continuously in a hypoxic chamber at 10% oxygen for three weeks with access to autoclaved drinking water, and the second is a control group in which the rats were housed under standard normoxic conditions (normoxia) for three weeks with access to autoclaved drinking water. The right ventricular systolic pressure and the ratio of RV to LV weight were determined at the end of the experimental period.

### (5-2) Administration of antibiotic cocktail to MCT

Six-week-old male SD rats were received subcutaneous injection of Crotaline (Sigma) at 60 mg/kg and were housed under standard normoxic conditions (normoxia) for three weeks with access to 3% sucrose drinking water containing four antibiotics (1 g/L ampicillin, 1 g/L fradiomycin, 1 g/L metronidazole and 0.5 g/L vancomycin) during the feeding period (MCT antibiotic group). As controls, two groups were constituted: the first is a MCT group in which the rats were received Crotaline administration and were housed under standard normoxic conditions (normoxia) for three weeks with access to sucrose drinking water, and the second is a control group in which the rats were housed under standard normoxic conditions (normoxia) for three weeks with access to sucrose drinking water without administration of Crotaline. The right ventricular systolic pressure and the ratio of RV to LV weight were determined at the end of the experimental period.

### (5-3) Administration of antibiotic cocktail to SuHx

Six-week-old male SD rats were received subcutaneous administration of Sugen 5416 and housed in a hypoxic chamber at 10% oxygen with access to autoclaved drinking water for three weeks. The rats were then transferred to and housed under standard normoxic conditions (normoxia) for two weeks with access to 3% sucrose drinking water containing four antibiotics (1 g/L ampicillin, 1 g/L fradiomycin, 1 g/L metronidazole and 0.5 g/L vancomycin) during the feeding period under standard normoxic conditions (SuHx antibiotic group). As controls, two groups were constituted: the first is a SuHx group in which the rats were received administration of Sugen 5416 and housed in a hypoxic chamber for three weeks and then transferred to and housed under standard normoxic conditions (normoxia) for two weeks with access to 3% sucrose drinking water during the feeding period under standard normoxic conditions, and the second is a control group in which the rats were housed under standard normoxic conditions (normoxia) without administration of Sugen 5416 for five weeks with access to autoclaved drinking water during the feeding period. The right ventricular systolic pressure and the ratio of RV to LV weight were determined at the end of the experimental period.

### (6) Statistical analysis

All data are expressed as the mean ± SEM. Significant difference tests among multiple groups were performed by one-way ANOVA followed by a post hoc comparison test with Scheffe's method. The Student's t-test was used to analyze differences between two groups. P < 0.05 was considered statistically significant.

### B. Results

### (1) Changes in gut microbiota

16S rRNA metagenomic analysis was performed on DNA extracted from the feces of the rat models of pulmonary hypertension induced by hypoxia (Hx), monocrotaline (MCT), or Sugen 5416/hypoxia/normoxia (SuHx) and non-treated rats (control group) to investigate the composition of the gut microbiota at the familial level. The results are shown in Fig. 2. All the rat models of pulmonary hypertension showed an increase in S24-7 and a decrease in Ruminococcaceae and Lachnospiraceae. The composition of the gut microbiota in AHRKO rats subjected to Sugen 5416/hypoxia/normoxia (SuHx) was closer to that of the non-treated rats (control group).

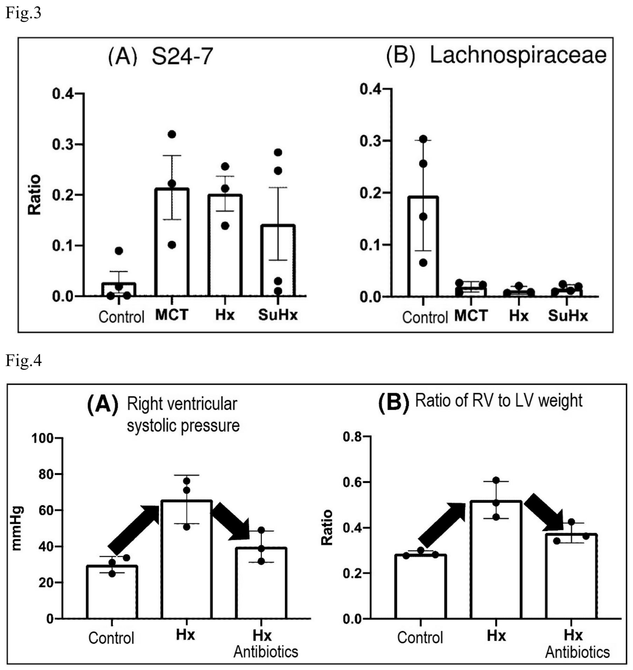

Fig. 3(A) shows the abundance of S24-7 in the gut microbiota in the control group, the Hx group, the MCT group and the SuHx group. All the rat models of pulmonary hypertension showed an increased abundance of S24-7 compared to that of the control group. Fig. 3(B) shows the abundance of Lachnospiraceae in the gut microbiota in the control group, the Hx group, the MCT group and the SuHx group. All the rat models of pulmonary hypertension showed a decreased abundance of Lachnospiraceae compared to that of the control group.

### (2) Effect of antibiotic-mediated gut microbiota intervention on the phenotypes of pulmonary hypertension (PH)

### (2-1) Hypoxia-induced PH model

The results are shown in Figs. 4(A) and 4(B). Fig. 4(A) shows the right ventricular systolic pressure, and Fig. 4(B) shows the ratio of RV to LV weight. The hypoxia-induced PH rat model not receiving the antibiotic cocktail (Hx group) showed an increase in the right ventricular systolic pressure and the ratio of RV to LV weight compared to those of the control group, indicating of the existence of the phenotypes of pulmonary hypertension. However, the hypoxia-induced PH rat model receiving the antibiotic cocktail (Hx antibiotic group) showed inhibition of increase in the right ventricular systolic pressure and the ratio of RV to LV weight, indicating that the phenotypes of pulmonary hypertension were improved.

### (2-2) Monocrotaline-induced PH model

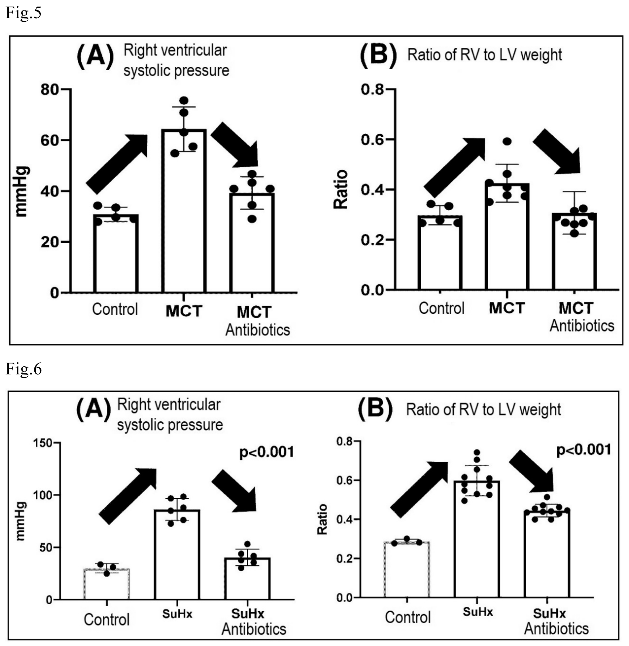

The results are shown in Figs. 5(A) and 5(B). Fig. 5(A) shows the right ventricular systolic pressure, and Fig. 5(B) shows the ratio of RV to LV weight. The monocrotaline-induced PH rat model not receiving the antibiotic cocktail (MCT group) showed an increase in the right ventricular systolic pressure and the ratio of RV to LV weight compared to those of the control group, indicating of the existence of the phenotypes of pulmonary hypertension. However, the monocrotaline-induced PH rat model receiving the antibiotic cocktail (MCT antibiotic group) showed inhibition of increase in the right ventricular systolic pressure and the ratio of RV to LV weight, indicating that the phenotypes of pulmonary hypertension were improved.

### (2-3) Sugen 5416/hypoxia/normoxia-induced PH model

The results are shown in Figs. 6(A) and 6(B). Fig. 6(A) shows the right ventricular systolic pressure, and Fig. 6(B) shows the ratio of RV to LV weight. The Sugen 5416/hypoxia/normoxia-induced PH rat model not receiving the antibiotic cocktail (SuHx group) showed an increase in the right ventricular systolic pressure and the ratio of RV to LV weight compared to those of the control group, indicating of the existence of the phenotypes of pulmonary hypertension. However, the Sugen 5416/hypoxia/normoxia-induced PH rat model receiving the antibiotic cocktail (SuHx antibiotic group) showed inhibition of increase in the right ventricular systolic pressure and the ratio of RV to LV weight, indicating that the phenotypes of pulmonary hypertension were improved.

### Example 2: Examination of gut microbiota in patients with pulmonary hypertension (1)

### A. Experimental materials and methods

### (1) Gut microbiota analysis by 16S rRNA metagenomic analysis

### (1-1) Subjects

Subjects included 25 patients with pulmonary hypertension and 25 healthy subjects. The patients with pulmonary hypertension included 11 patients with idiopathic or hereditary pulmonary arterial hypertension (PAH), 4 patients with collagen tissue disease-associated pulmonary hypertension (PH), 1 patient with portal hypertension-associated PH, 2 patients with drug-induced PH, 3 patients with chronic thromboembolic PH, and 4 patients with congenital shunt disease-associated PH.

### (1-2) Metagenomic analysis of 16S rRNA

16S rRNA metagenomic analysis was performed in the same manner as in Example 1.

### (1-3) Evaluation of alpha diversity

Alpha diversity was assessed by Shannon index and Chao index. These indexes indicate the number of different bacteria that are present in a fecal sample and the composition of the bacterial communities as well as the degree of bias in the phylogenetic relationship between different bacteria. The indexes were calculated using QIIME version 1.9.1.

### (1-4) Evaluation of beta diversity

Beta diversity was assessed by principal component analysis to investigate the composition of the bacterial communities and their abundance in individuals to estimate the difference in the composition of the gut bacterial communities between individuals. The beta diversity metrics were calculated using QIIME version 1.9.1.

### (1-5) Measurement of the activity of aryl hydrocarbon receptor (AHR)

Luciferase reporter assay was performed using Human AhR Reporter Assay System (INDIGO Biosciences) to measure the serum AHR activity. The assay was performed according to the manufacturer's instructions, except for stimulation conditions. Stimulation was performed by adding the serum to culture medium as specified in the manufacturer's instructions at a final concentration of 10%.

### (2) Gut microbiota analysis by whole metagenomic analysis

### (2-1) Subjects

Subjects included 5 patients with pulmonary hypertension and 5 healthy subjects.

### (2-2) Whole metagenomic analysis

Whole genome sequencing was performed on DNBSEQ-G400 (MGI Tech) to generate 150 bp paired-end reads. Low quality reads were excluded using fastp version 0.20.0. The host-derived reads corresponding to human reference genome data (GRCh38) were excluded using bowtie2 version 2.3.5. The composition of bacterial communities was identified using MetaPhlAn2 version 2.6.0. and heat maps were generated using hclust2.py.

### (3) Functional analysis of gut microbiota

The functional metabolic profile of the gut microbiota was analyzed by HUMAnN2 version 0.9.9.

### (4) Measurement of IgA levels in feces

Fecal samples were weighed, and diluted in PBS to prepare 1000-fold diluted suspensions. The IgA levels in the suspensions were measured using Human IgA ELISA kit (Bethyl Laboratories Inc.). The IgA levels in the fecal samples of rats were also determined in the same manner as above using Rat IgA ELISA Kit (GenWay).

### (5) Statistical analysis

Statistical analysis was performed in the same manner as in Example 1.

### B. Results

### (1) Results of 16S rRNA metagenomic analysis of DNA in feces

### (1-1) Changes in gut microbiota as assessed by 16S rRNA metagenomic analysis of

### DNA in feces

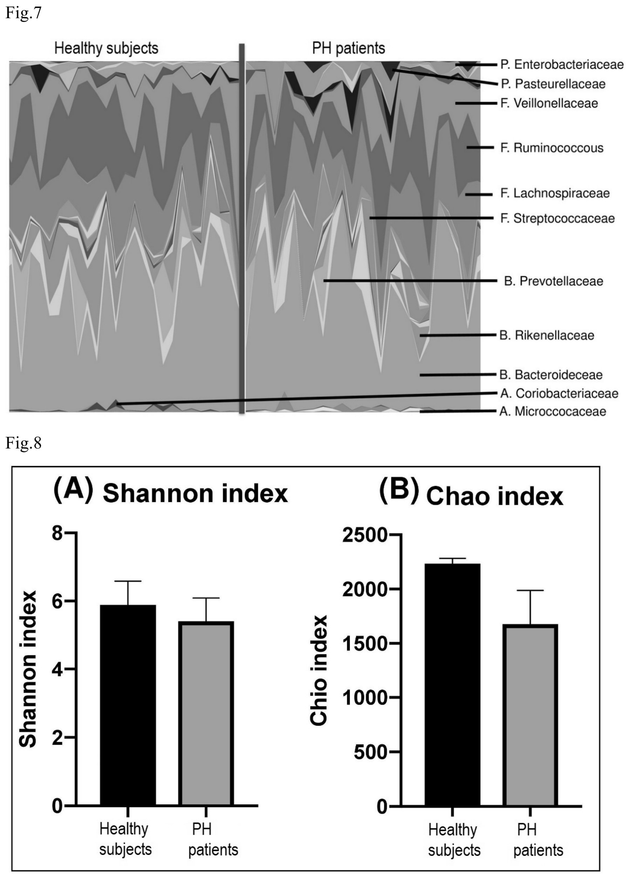

16S rRNA metagenomic analysis of DNA in the fecal samples of the pulmonary hypertension (PH) patients and the healthy subjects was performed to analyze the composition of the gut microbiota at the familial level. The results are shown in Fig. 7. The PH patients showed an increase in some of the gut bacteria (Streptococcaceae, Micrococcaceae, Pasteurellaceae, Veillonellaceae, etc.) compared to the healthy subjects. The PH patients also showed a decrease in some of the gut bacteria (Rikenellaceae, Ruminococcaceae, Coriobacteriaceae, etc.) compared to the healthy subjects.

### (1-2) Evaluation of alpha diversity

The results are shown in Figs. 8(A) and 8(B). Fig. 8(A) shows the Shannon index and Fig. 8(B) shows the Chao index. These indexes were significantly decreased in the PH patients compared to those in the healthy subjects.

### (1-3) Evaluation of beta diversity

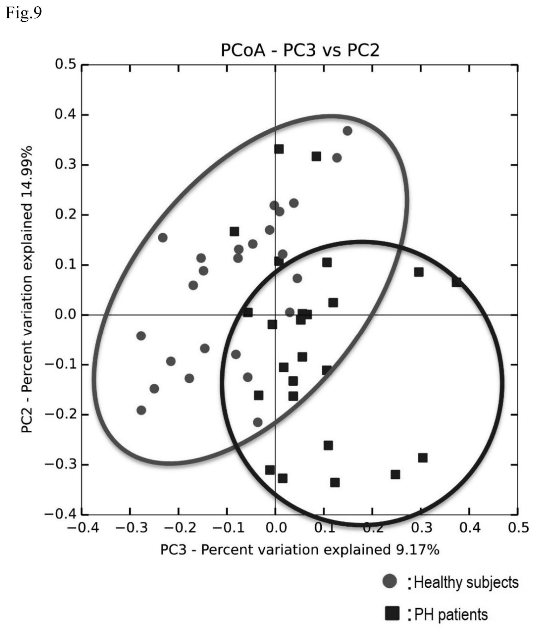

The results of principal component analysis are shown in Fig. 9. The healthy subjects and the PH patients were clustered differently, indicating that the composition of the gut microbiota in the PH patients is clearly different from that of the healthy subjects.

### (1-4) Detailed examination of gut bacteria that are increased in PH patients

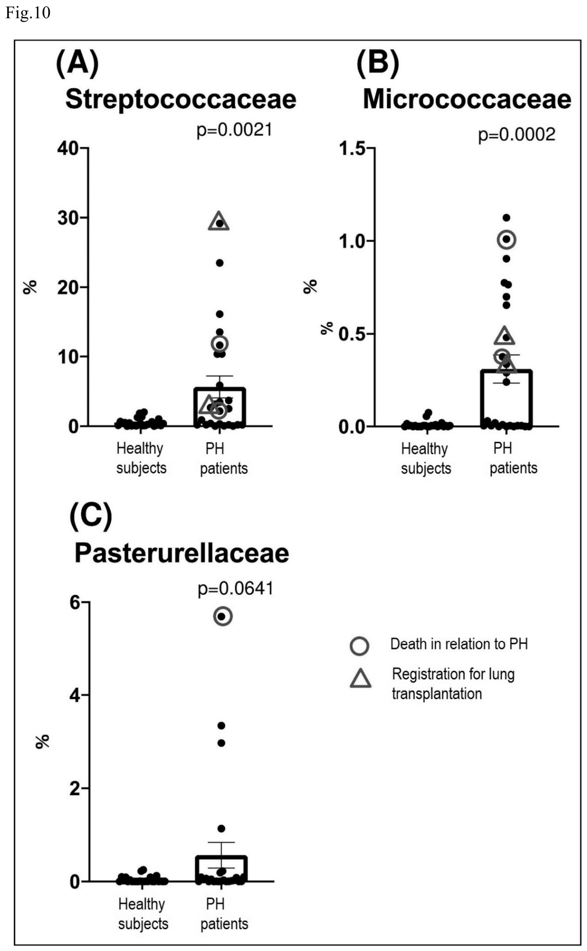

The abundance of the bacteria belonging to the family (A) Streptococcaceae, (B) Micrococcaceae or (C) Pasteurellaceae was plotted for individual healthy subjects and PH patients in Figs. 10(A) to 10(C) to compare the mean value of the abundance of the bacteria between the groups. The figures also indicate the PH patients who died in relation to pulmonary hypertension after this study and the PH patients who registered for lung transplantation after this study. All the bacteria (bacterial families) were significantly increased in the PH patient group compared to those in the healthy subject group. The PH patients with a high abundance of these bacterial families are at a high risk of death in relation to pulmonary hypertension or need to register for lung transplantation.

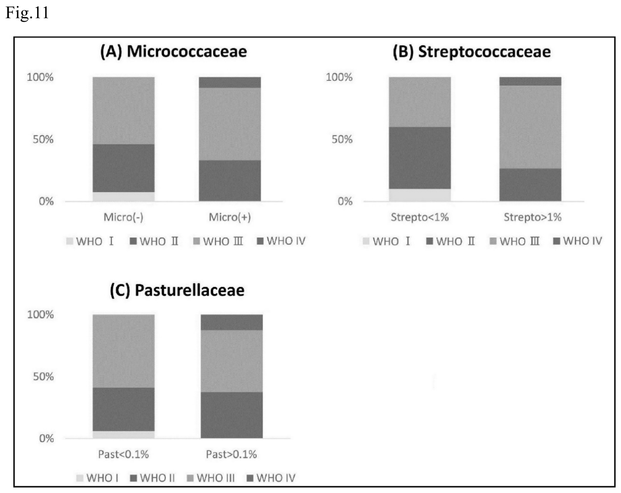

The relation between the changes in the gut bacteria and the severity of pulmonary hypertension was investigated in the PH patients and is shown in Figs. 11(A) to 11(C). The severity of pulmonary hypertension was determined based on the WHO Functional Classification of Pulmonary Hypertension (Table 2). Fig. 11(A) shows the comparison of the severity of pulmonary hypertension between the PH patients in which Micrococcaceae bacteria were not detected and the PH patients in which Micrococcaceae bacteria were detected. Fig. 11(B) shows the comparison of the severity of pulmonary hypertension between the PH patients in which Streptococcaceae bacteria were detected with an abundance of less than 1% and the PH patients in which Streptococcaceae bacteria were detected with an abundance of 1% or more. Fig. 11(C) shows the comparison of the severity of pulmonary hypertension between the PH patients in which Pasteurellaceae bacteria were detected with an abundance of less than 0.1% and the PH patients in which Pasteurellaceae bacteria were detected with an abundance of 0.1% or more. As apparent from Fig. 11(A), pulmonary hypertension was more severe in the PH patients in which Micrococcaceae bacteria were detected. Also shown in Fig. 11(B), pulmonary hypertension was more severe in the PH patients in which Streptococcaceae bacteria were detected with an abundance of 1% or more. Further shown in Fig. 11(C), pulmonary hypertension was more severe in the PH patients in which Pasteurellaceae bacteria were detected with an abundance of 0.1% or more. Overall results revel that the changes in the gut microbiota in the PH patients reflect the severity of pulmonary hypertension. Therefore, the examination of gut bacteria in PH patients can be used to assist the determination of the severity of the PH patients.

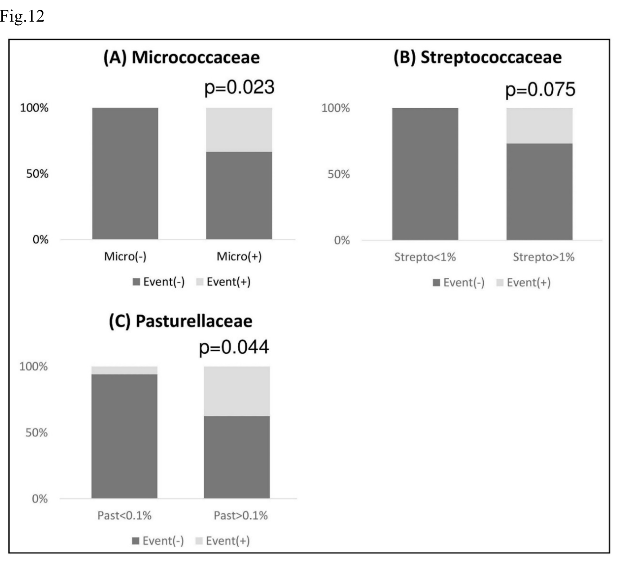

The relation between the changes in the gut bacteria and the prognosis of pulmonary hypertension was investigated in the PH patients and is shown in Figs. 12(A) to 12(C). The prognostic events investigated in this study were death in relation to pulmonary hypertension and registration for lung transplantation. Fig. 12(A) shows the comparison of the occurrence of the events between the PH patients in which Micrococcaceae bacteria were not detected and the PH patients in which Micrococcaceae bacteria were detected. Fig. 12(B) shows the comparison of the occurrence of the events between the PH patients in which Streptococcaceae bacteria were detected with an abundance of less than 1% and the PH patients in which Streptococcaceae bacteria were detected with an abundance of 1% or more. Fig. 12(C) shows the comparison of the occurrence of the events between the PH patients in which Pasteurellaceae bacteria were detected with an abundance of less than 0.1% and the PH patients in which Pasteurellaceae bacteria were detected with an abundance of 0.1% or more. As apparent from Fig. 12(A), the events occurred only in the PH patients in which Micrococcaceae bacteria were detected. Also shown in Fig. 12(B), the events occurred only in the PH patients in which Streptococcaceae bacteria were detected with an abundance of 1% or more. Further shown in Fig. 12(C), the number of patients who underwent the events was significantly larger in the PH patients in which Pasteurellaceae bacteria were detected with an abundance of 0.1% or more. Therefore, the examination of gut bacteria in PH patients can be used to predict the prognosis of the PH patients.

The relation between the changes in the gut bacteria and the serum AHR activity in the PH patients was investigated. Fig. 13(A) shows the measurement results of the serum AHR activity in the healthy subjects and the PH patients. The serum AHR activity was significantly higher in the PH patients than in the healthy subjects. The PH patients were then classified into patient groups with severity 1-2 or 3-4 according to the WHO Functional Classification of Pulmonary Hypertension (Table 2). The serum AHR activity in the patient groups and the healthy subject group was measured and the results of the measurement are shown in Fig. 13(B). As apparent from the figure, when the severity is higher, the serum AHR activity is significantly higher. Fig. 13(C) shows the correlation between the pulmonary vascular resistance (PVR) and the serum AHR activity in the PH patients. Significant positive correlation was observed between the PVR and the serum AHR activity in the PH patients. Overall results indicate that the serum AHR activity correlates with the severity of the PH patients.

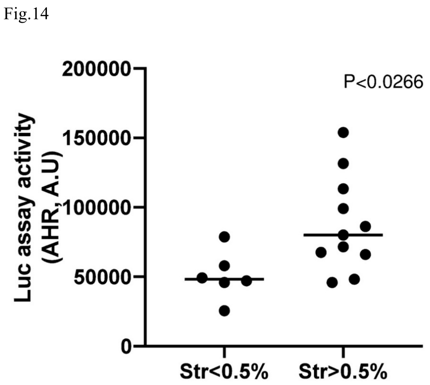

The serum AHR activity was compared between the PH patients in which Streptococcaceae bacteria were detected with an abundance of less than 0.5% and the PH patients in which Streptococcaceae bacteria were detected with an abundance of 0.5% or more. The results are shown in Fig. 14. The serum AHR activity was significantly higher in the PH patients in which Streptococcaceae bacteria were detected with an abundance of 0.5% or more than in the PH patients in which Streptococcaceae bacteria were detected with an abundance of less than 0.5%. These results further support the finding that the examination of gut bacteria in PH patients can be used to assist the determination of the severity of the PH patients.

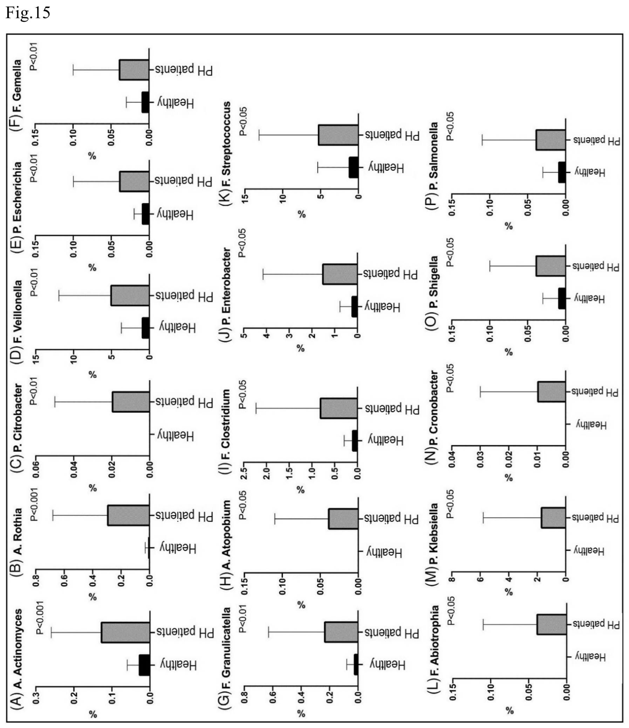

The gut bacteria that were increased in the PH patients were compared to those in the healthy subjects at the genus level. The results are shown in Figs. 15(A) to 15(P). The gut bacteria that were significantly increased in the PH patient group as compared to those in the healthy subject group were bacteria belonging to the genus (A) *Actinomyces,* (B) *Rothia,* (C) *Citrobacter,* (D) *Veillonella,* (E) *Escherichia,* (F) *Gemella,* (G) *Granulicatella,* (H) *Atopobium,* (I) *Clostridium,* (J) *Enterobacter,* (K) *Streptococcus,* (L) *Abiotrophia,* (M) *Klebsiella,* (N) *Cronobacter,* (O) *Shigella* and (P) *Salmonella.*

### (1-5) Detailed examination of gut bacteria that are decreased in PH patients

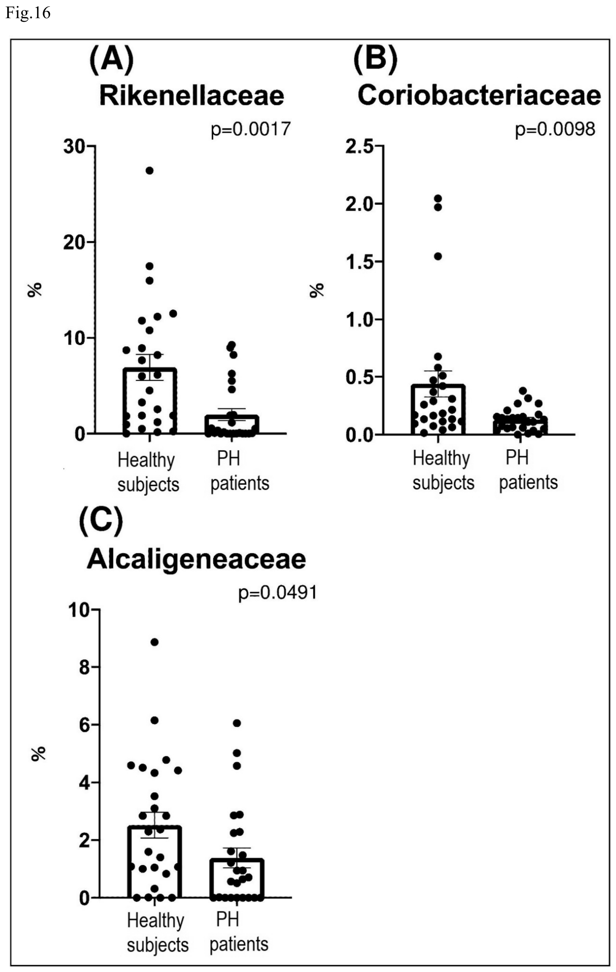

The abundance of the bacteria belonging to the family (A) Rikenellaceae, (B) Coriobacteriaceae or (C) Alcaligenaceae was plotted for individual healthy subjects and PH patients in Figs. 16(A) to 16(C) to compare the mean value of the abundance of the bacteria between the groups. All the bacteria (bacterial families) were significantly decreased in the PH patient group compared to those in the healthy subjects.

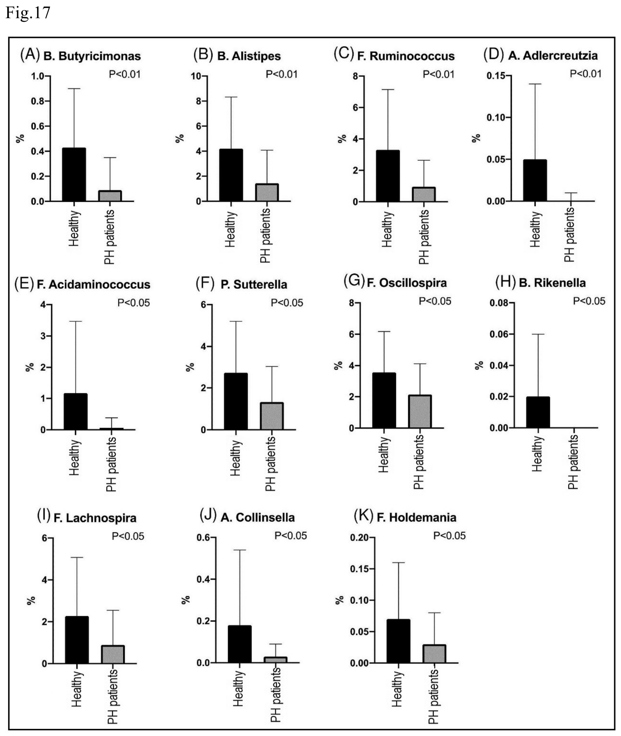

The gut bacteria that were decreased in the PH patients were compared to those in the healthy subjects at the genus level. The results are shown in Figs. 17(A) to 17(K). The gut bacteria that were significantly decreased in the PH patient group as compared to those in the healthy subject group were bacteria belonging to the genus (A) *Butyricimonas,* (B) *Alistipes,* (C) *Ruminococcus,* (D) *Adlercreutzia,* (E) *Acidaminococcus,* (F) *Sutterella,* (G) *Oscillospira,* (H) *Rikenella,* (I) *Lachnospira,* (J) *Collinsella* and (K) *Holdemania.*

### (2) Results of whole metagenomic analysis of DNA in feces

### (2-1) Changes in gut microbiota as assessed by whole metagenomic analysis of DNA in feces

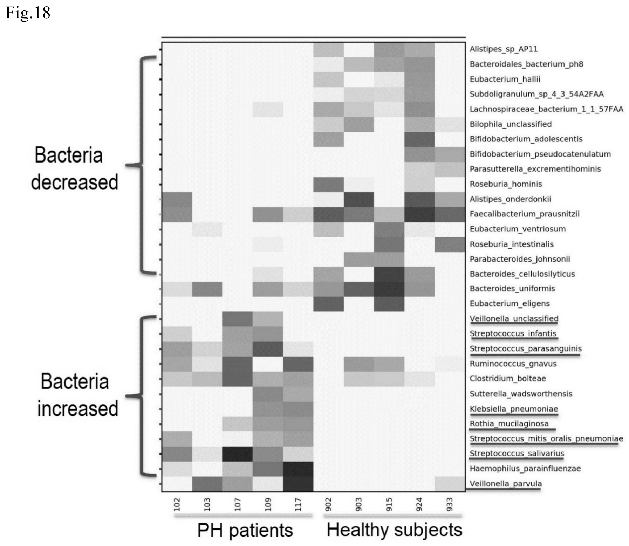

Whole metagenomic analysis of DNA in the fecal samples of the PH patients and the healthy subjects was performed. The results are shown in Fig. 18. The gut bacteria that were increased in the PH patients were *Veillonella*_unclassified, *Streptococcus*_*infantis, Streptococcus_parasanguinis, Ruminococcus_gnavus, Clostridium_bolteae, Sutterella_wadsworthensis, Klebsiella_pneumoniae, Rothia_mucilaginosa, Streptococcus_mitis_oralis_pneumoniae, Streptococcus_salivarius, Haemophilus_parainfluenzae,* and *Veillonella_parvula.* These bacteria except for *Ruminococcus_gnavus, Clostridium_bolteae, Sutterella_wadsworthensis* and *Haemophilus_parainfluenzae* were normal flora bacteria in the oral cavity (the bacteria indicated by the underline in the figure). The gut bacteria that were decreased in the PH patients were *Alistipes*_sp_AP11, Bacteroidales_bacterium, *Eubacterium_hallii, Subdoligranulum_*sp_4_3_54A2FAA, Lachnospiraceae_bacterium_1_1_57FAA, *Bilophila_unclassified, Bifidobacterium_adolescentis, Bifidobacterium_pseudocatenulatum, Parasutterella_excrementihominis, Roseburia_hominis, Alistipes_onderdonkii, Faecalibacterium_prausnitzii, Eubacterium*_*ventriosum, Roseburia_intestinalis, Parabacteroides_johnsonii, Bacteroides_cellulosilyticus, Bacteroides_uniformis* and *Eubacterium_eligens.*

### (2-2) Functional analysis of gut microbiota

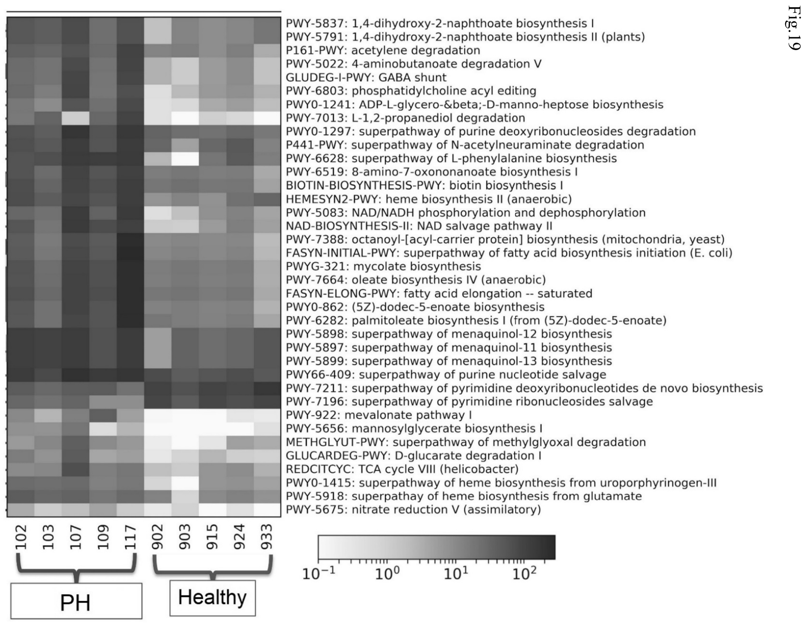

The functional metabolic profile of the gut microbiota was analyzed using the data generated by the whole metagenomic analysis. The results are shown in Fig. 19. The analysis revealed that the functions relating to the mevalonate pathway, the mannosylglycerate synthesis pathway, the methylglyoxal degradation pathway, the D-glucarate degradation pathway, the TCA cycle VIII pathway, the heme biosynthesis pathway and the nitrate metabolism pathway were enhanced in the PH patients.

### (3) IgA levels in feces

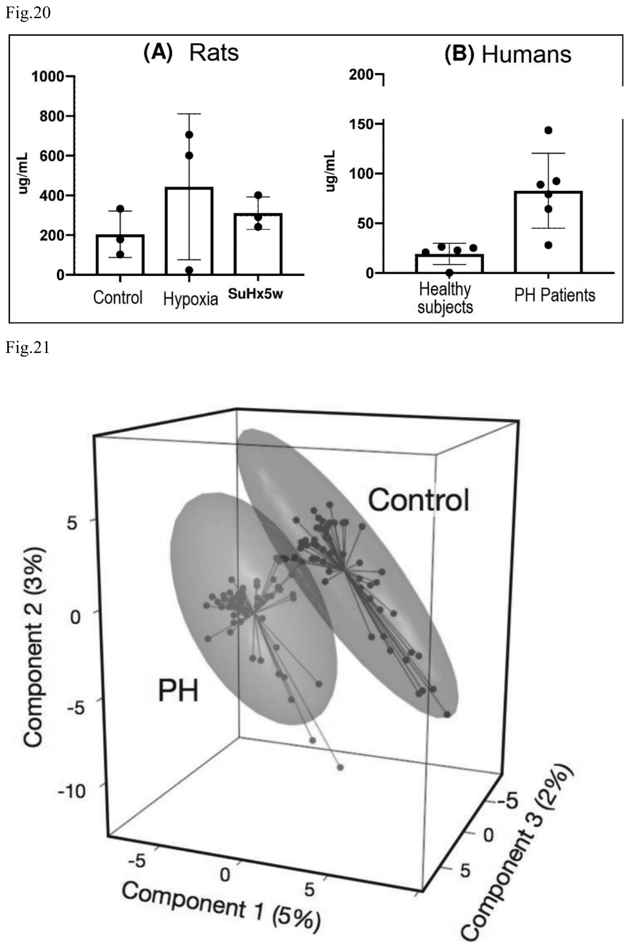

The results are shown in Figs. 20(A) and 20(B). Fig. 20(A) shows the IgA levels in the fecal samples of rats, and Fig. 20(B) shows the IgA levels in the fecal samples of humans. For rats, the IgA levels in the fecal samples were increased in the hypoxia-induced PH rat model (Hx group) and the Sugen 5416/hypoxia/normoxia-induced PH rat model (SuHx group) compared to those in the control group. For humans, the IgA levels in the fecal samples were increased in the PH patient group compared to those in the healthy subject group.

### Example 3: Examination of gut microbiota in patients with pulmonary hypertension (2)

### A. Experimental materials and methods

### (1) Subjects

Subjects included 57 patients with pulmonary hypertension and 57 healthy subjects. The 57 patients included 25 patients with idiopathic or hereditary pulmonary arterial hypertension (I/HPAH), 12 patients with collagen tissue disease-associated pulmonary arterial hypertension (CTD-PAH), 6 patients with congenital shunt disease-associated pulmonary arterial hypertension (CHD-PAH), 3 patients with portal hypertension-associated pulmonary hypertension (PoPH), 4 patients with chronic thromboembolic pulmonary hypertension (CTEPH), and 7 patients with other types of pulmonary hypertension (drug-induced pulmonary hypertension etc.).

### (2) Metagenomic analysis of 16S rRNA

16S rRNA metagenomic analysis was performed in the same manner as in Example 1. The Linear Discriminant Analysis (LDA) scores were computed under the Bioconda environment to determine the biological features of the 16S rRNA metagenomic information of the gut microbiota in the PH patients and the healthy subjects. A cladogram was generated based on LDA effect size (LEfSe) analysis to determine the phylogenetic features of the gut microbiota in the PH patients. Composite events were defined to include death, lung transplantation, and hospitalization with right-sided heart failure. Random forest analysis was used to rank the gut bacteria (at the genus level) that are associated with the occurrence of the composite events to narrow down the gut bacteria that are strongly associated with the composite events (death, lung transplantation, and hospitalization with right-sided heart failure).

### (3) Measurement of serum IL-6 levels

The IL-6 levels in the serum of the patients were measured using IL-6 ELISA kit (R&D systems). The collection of blood was performed at the same time as collecting the fecal samples.

### B. Results

### (1) Results of 16S rRNA metagenomic analysis of DNA in feces

### (1-1) Partial least-square discriminant analysis (PLS-DA)

The results of PLS-DA analysis are shown in Fig. 21. PLS-DA means partial least-square discriminant analysis. In this study, PLS-DA analysis was conducted using the information on the frequency of the gut bacteria as an explanatory variable to classify the healthy subjects and the PH patients, and the scores were plotted in a three dimensional space to visualize them. In the figure, Control indicates the healthy subjects, and PH indicates the PH patients. The PLS-DA identifies a clear clustering between the healthy subjects and the PH patients.

### (1-2) Alpha diversity

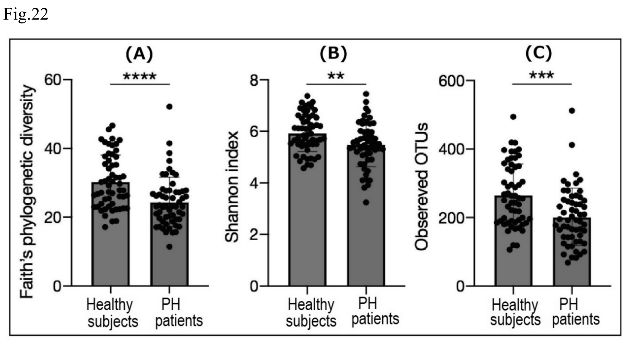

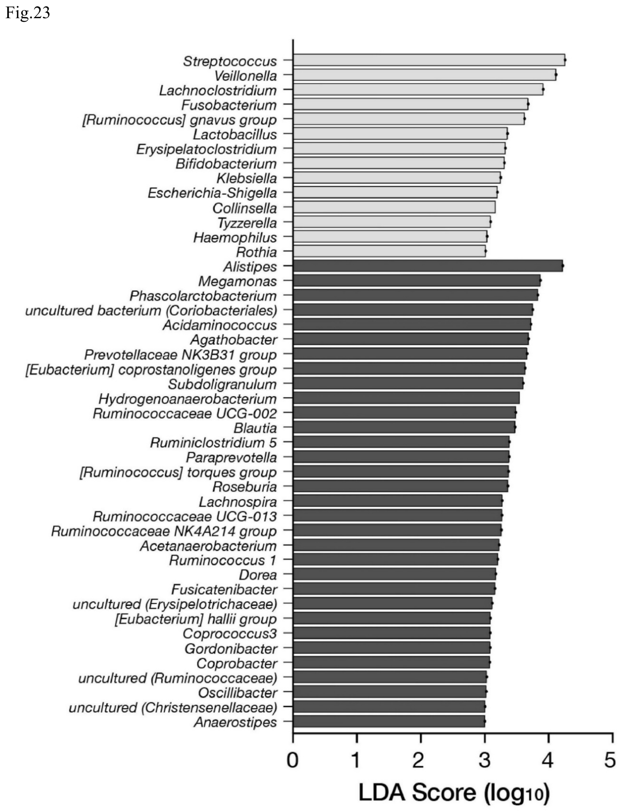

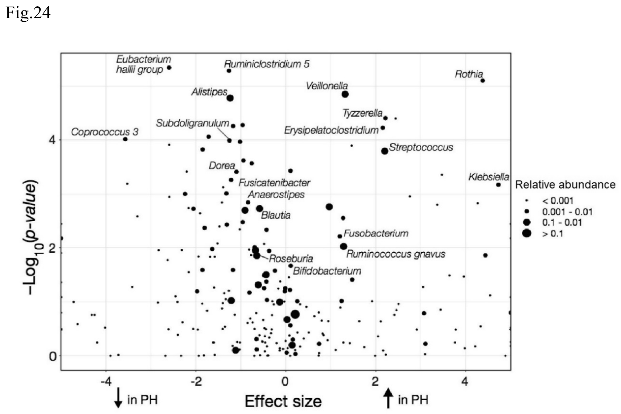

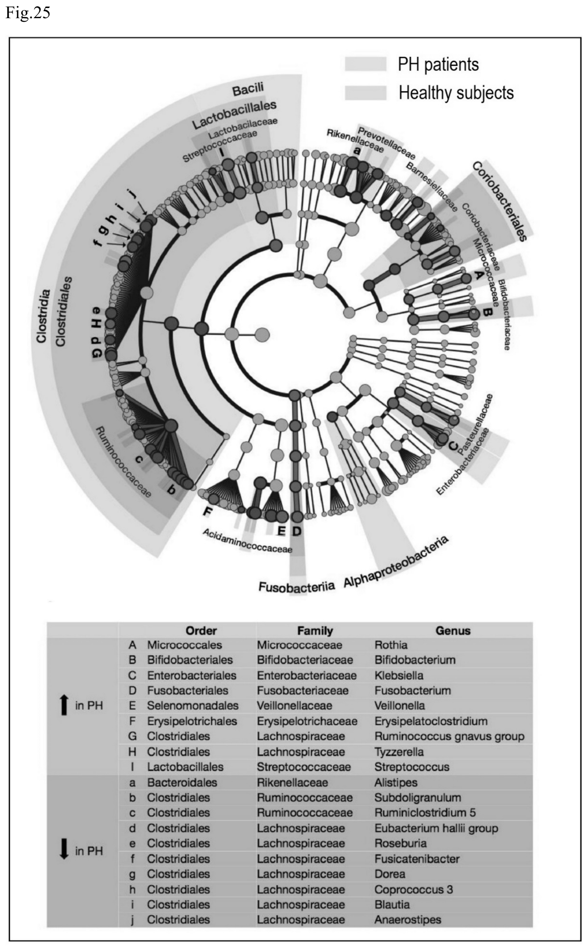

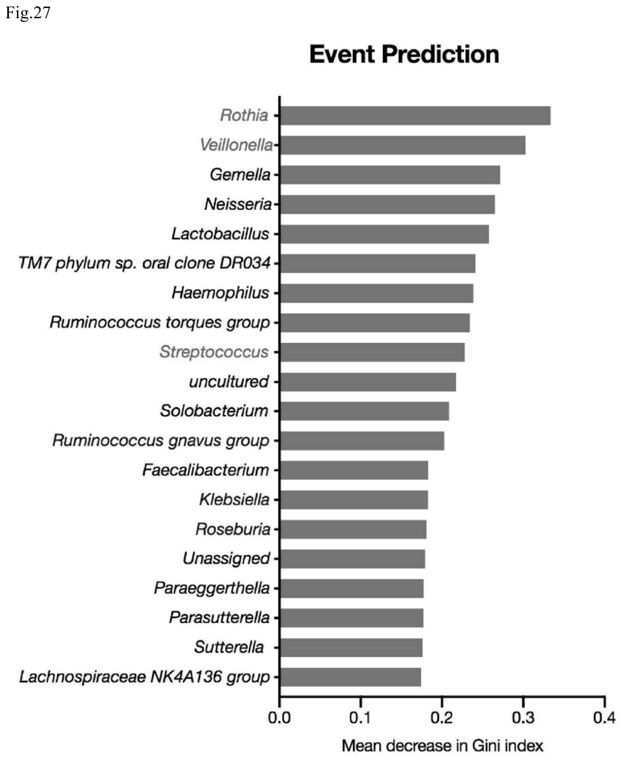

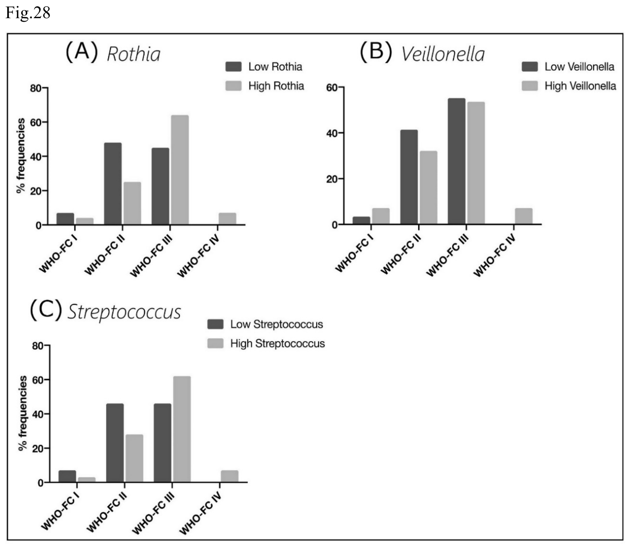

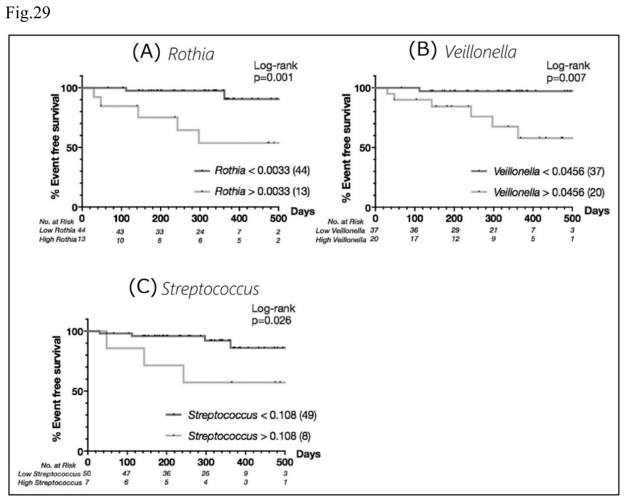

The results of alpha diversity analysis are shown in Figs. 22(A) to 22(C). Fig. 22(A) shows the Faith's phylogenetic diversity. Fig. 22(B) shows the Shannon index. Fig. 22(C) shows the observed OTUs. All the indexes were significantly decreased in the PH patients compared to those in the healthy subjects.
**(1-3)**
   The gut bacteria that were statistically significantly increased or decreased in the PH patients were determined (at the genus level) using a LDA score of 3.0 or more as a cut-off value. The results are shown in Fig. 23. The bacteria that were increased in the PH patients were *Streptococcus, Veillonella, Lachnoclostridium, Fusobacterium,* [*Ruminococcus*] *gnavus* group, *Lactobacillus, Erysipelatoclostridium, Bifidobacterium, Klebsiella, Escherichia-Shigella, Collinsella, Tyzzerella, Haemophilus* and *Rothia.* The bacteria that were decreased in the PH patients were *Alistipes, Megamonas, Phascolarctobacterium,* uncultured bacterium (Coriobacteriales), *Acidaminococcus, Agathobacter,* Prevotellaceae NK3B31 group, [*Eubacterium*] *coprostanoligenes* group, *Subdoligranulum, Hydrogenoanaerobacterium, Ruminococcaceae* UCG-002, *Blautia, Ruminiclostridium 5, Paraprevotella, [Ruminococcus] torques* group, *Roseburia,* Ruminococcaceae UCG-013, *Lachnospira,* Ruminococcaceae NK4A214 group, *Acetanaerobacterium, Ruminococcus 1, Dorea, Fusicatenibacter,* uncultured (Erysipelotrichaceae), [*Eubacterium*] *hallii* group, *Coprococcus 3, Gordonibacter, Coprobacter,* uncultured (Ruminococcaceae), *Oscillibacter,* uncultured (Christensenellaceae) and *Anaerostipes.*
**(1-4)**
   To identify the gut bacteria that were more significantly changed, a volcano plot was generated using significant differences in changes (-Log₁₀ (p-value)) against the fold change (Effect size) in the PH patients and is shown in Fig. 24. The dot sizes reflect the relative abundance of the gut bacteria at the genus level that were present in the healthy subjects and the PH patients.
**(1-5)**
   A cladogram was generated based on the LDA scores and the volcano plot to identify the gut bacteria that were significantly changed at the genus level. The cladogram is shown in Fig. 25. The gut bacteria that were predominantly increased were *Rothia* bacteria belonging to the family Micrococcaceae, *Bifidobacterium* bacteria belonging to the family Bifidobacteriaceae, *Fusobacterium* bacteria belonging to the family Fusobacteriaceae, *Veillonella* bacteria belonging to the family Veillonellaceae, *Erysipelatoclostridium* bacteria belonging to the family Erysipelotrichaceae, *Ruminococcus gnavus* group belonging to the family Lachnospiraceae, *Tyzzerella* bacteria belonging to the family Lachnospiraceae, and *Streptococcus* bacteria belonging to the family Streptococcaceae. Most of them were normal flora bacteria in the oral cavity. The gut bacteria that were predominantly decreased were *Alistipes* bacteria belonging to the family Rikenellaceae, *Subdoligranulum* bacteria belonging to the family Ruminococcaceae, *Ruminiclostridium* 5 bacteria belonging to the family Ruminococcaceae, *Eubacterium hallii* group belonging to the family Lachnospiraceae, *Roseburia* bacteria belonging to the family Lachnospiraceae, *Fusicatenibacter* bacteria belonging to the family Lachnospiraceae, *Dorea* bacteria belonging to the family Lachnospiraceae, *Coprococcus* bacteria belonging to the family Lachnospiraceae, *Blautia* bacteria belonging to the family Lachnospiraceae, and *Anaerostipes* bacteria belonging to the family Lachnospiraceae. Most of them belong to *Clostridium* cluster IV/XIVa.
**(1-6)**
   The frequencies of 18 different bacteria (at the genus level) that were increased or decreased were compared between the subgroups of pulmonary hypertension. The results are shown in Figs. 26(A) to 26(R). The frequencies are shown respectively in (A) *Rothia,* (B) *Bifidobacterium,* (C) *Klebsiella,* (D) *Veillonella,* (E) *Erysipelatoclostridium,* (F) *Ruminococcus gnavus* group, (G) *Tyzzerella,* (H) *Streptococcus,* (I) *Alistipes,* (J) *Subdoligranulum,* (K) *Ruminiclostridium 5,* (L) *Eubacterium hallii* group, (M) *Roseburia,* (N) *Fusicatenibacter,* (O) *Dorea,* (P) *Coprococcus,* (Q) *Blautia* and (R) *Anaerostipes.* In the figures, Control indicates the healthy subjects, I/HPAH indicates idiopathic or hereditary pulmonary arterial hypertension, CTD-PAH indicates collagen tissue disease-associated pulmonary arterial hypertension, CHD-PAH indicates congenital shunt disease-associated pulmonary arterial hypertension, PoPH indicates portal hypertension-associated pulmonary hypertension, CTEPH indicates chronic thromboembolic pulmonary hypertension, and Others indicates other types of pulmonary hypertension (including drug-induced pulmonary hypertension etc.). (B) *Bifidobacterium,* (O) *Dorea* and (Q) *Blautia* were significantly and specifically increased in portal hypertension-associated PH. (C) *Klebsiella* bacteria were significantly and specifically increased in chronic thromboembolic PH.
**(1-7)**
   The gut bacteria that are associated with the occurrence of the pulmonary hypertension-related events (composite events including right-sided heart failure, lung transplantation, and death) were ranked by random forest analysis. The results are shown in Fig. 27. The results indicate that the *Rothia, Streptococcus, Haemophilus* and *Veillonella* bacteria are associated with the occurrence of the composite events.
**(1-8)**
   Receiver operating characteristics curve (ROC) analysis was performed to calculate an optimal cut-off value for prediction of the occurrence of the composite events. The patients were divided into two groups using the calculated cut-off value that most accurately predicts the occurrence of the composite events, and the relation between the frequencies of the gut bacteria and the severity of pulmonary hypertension was analyzed (see the WHO Functional Classification of Pulmonary Hypertension in Table 2). The results are shown in Figs. 28(A) to 28(C). The relation between the frequencies of the gut bacteria and the severity of pulmonary hypertension is shown in Figs. 28(A) *Rothia,* (B) *Veillonella* and (C) *Streptococcus.* The severity classified according to the WHO Functional Classification of Pulmonary Hypertension was significantly greater in the patients having the gut bacteria containing > 0.33% *Rothia* bacteria and > 10.8% *Streptococcus* bacteria.
**(1-9)**
   The patients were divided into two groups based on the prediction of the occurrence of the pulmonary hypertension-related events using the cut-off values for *Rothia, Veillonella* and *Streptococcus,* and the event-free survival was compared using the Kaplan-Meier test. The results are shown in Figs. 29(A) to 29(C). The event-free survival is shown in Figs. 29(A) *Rothia,* (B) *Veillonella* and (C) *Streptococcus.* The patient group having the gut bacteria containing > 0.33% *Rothia* bacteria, > 4.56% *Veillonella* bacteria and >10.8% *Streptococcus* bacteria had a more frequent occurrence of the composite events, including death, right-sided heart failure and lung transplantation, compared to the patient group having the gut bacteria containing < 0.33% *Rothia* bacteria, < 4.56% *Veillonella* bacteria and < 10.8% *Streptococcus* bacteria.

### (2) Correlation between gut bacteria and serum IL-6 levels

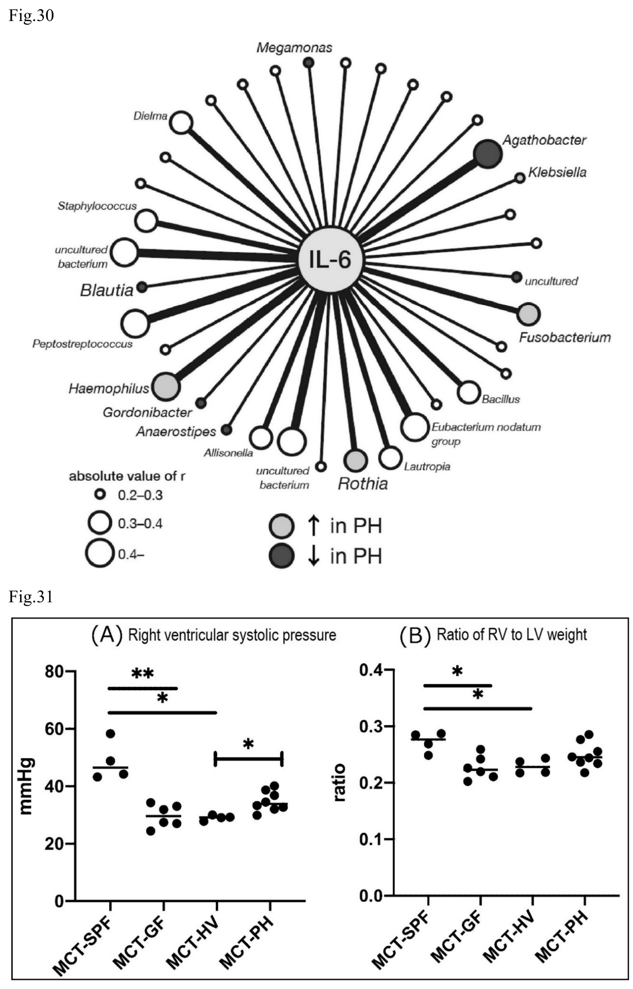

The correlation coefficient between the relative abundance of the gut bacteria (at the genus level) and the serum IL-6 levels was calculated using a round-robin approach. The network analysis of the gut bacteria using a correlation coefficient (r) of > 2 is shown in Fig. 30. The relative abundance of the bacteria belonging to the genus *Haemophilus, Klebsiella, Fusobacterium* and *Rothia* was correlated with the serum IL-6 levels quantified by ELISA.

### Example 4: Examination of gut microbiota in gnotobiotic rat model of pulmonary hypertension

### A. Experimental materials and methods

### (1) Preparation of gnotobiotic rat model of pulmonary hypertension

Germ-free F344 male rats were purchased from Japan SLC, Inc. and housed in an aseptic isolator. The feces of healthy subjects or PH patients were administered to the rats at the age of 11 weeks, and monocrotaline was administered to the rats at the age of 15 weeks to prepare a gnotobiotic rat model of pulmonary hypertension. Specifically, the feces of four healthy subjects were mixed in an anaerobic chamber, and diluted in an anaerobic transport medium (in a volume of 1000 mL containing 20 g of Lab-Lemco powder, 1 g of L-cysteine, 0.45 g of KH₂PO₄, 0.9 g of NaCl, 0.45 g of (NH₄)₂SO₄, 0.045 g of CaCl₂, 0.045 g of MgSO₄, 400 mL of glycerol, and 600 mL of distilled water) to prepare a dilution of the feces of the healthy subjects. The feces of four patients were mixed in an anaerobic chamber and diluted in the anaerobic transport medium in the same manner as above to prepare a dilution of the feces of the patients. The fecal dilution of the healthy subjects or the patients was orally administered to the rats at the age of 11 weeks in separate isolators to prepare gnotobiotic rats that simulate the gut microbiota in the healthy subjects or the patients. Four weeks after the fecal transplantation (at the age of 15 weeks), 60 mg/kg of monocrotaline was subcutaneously injected, and the rats were housed in an isolator for three weeks to prepare a gnotobiotic rat model of pulmonary hypertension induced by administration of monocrotaline.

### (2) Control animals

To germ-free F344 male rats at the age of 15 weeks not receiving a dilution of feces, 60 mg/kg of monocrotaline was subcutaneously injected, and the rats were housed in an isolator for three weeks to prepare a germ-free F344 rat model of pulmonary hypertension induced by administration of monocrotaline. These rats were used as control rats. As another control group, F344 male rats were purchased from Japan SLC, Inc. and housed in an SPF environment at the National Cerebral and Cardiovascular Center. Subcutaneous injection of 60 mg/kg of monocrotaline was given to the rats at the age of 15 weeks, and the rats were housed in the SPF environment for three weeks to prepare a SPF F344 rat model of pulmonary hypertension induced by administration of monocrotaline.

The right ventricular systolic pressure and the right ventricular hypertrophy were determined for all the rats at the age of 18 weeks. Measurement of the right ventricular systolic pressure was performed in the same manner as in the section (2) in A. Experimental materials and methods in Example 1. Measurement of the right ventricular hypertrophy was performed in the same manner as in the section (3) Ratio of right ventricle to left ventricle weight in A. Experimental materials and methods.

### B. Results

The results are shown in Figs. 31(A) and 31(B). Fig. 31(A) shows the right ventricular systolic pressure, and Fig. 31(B) shows the ratio of RV to LV weight (the right ventricular hypertrophy). In the figures, MCT-SPF indicates the F344 rats housed in the SPF environment, MTC-GF indicates the F344 rats housed in the germ-free environment, MTC-HV indicates the F344 rats receiving fecal transplantation from the healthy subjects, and MTC-PH indicates the F344 rats receiving fecal transplantation from the patients. The F344 rats housed in the germ-free environment showed a significant decrease in the right ventricular systolic pressure and the ratio of RV to LV weight compared to those of the F344 rats housed in the SPF environment, indicating that monocrotaline-induced pulmonary hypertension was significantly inhibited. The F344 rats receiving fecal transplantation from the healthy subjects showed a comparable right ventricular systolic pressure and a comparable ratio of RV to LV weight to those of the F344 rats housed in the germ-free environment, indicating that monocrotaline-induced pulmonary hypertension was significantly inhibited. The F344 rats receiving fecal transplantation from the patients showed a significant increase in the right ventricular systolic pressure compared to that of the F344 rats receiving fecal transplantation from the healthy subjects.

### Example 5: Measurement of short-chain fatty acid levels in human feces

The acetic acid, propionic acid and butyric acid levels in the feces of healthy subjects and PH patients were measured by high performance liquid chromatography (Shimadzu Organic Acid Analysis System).

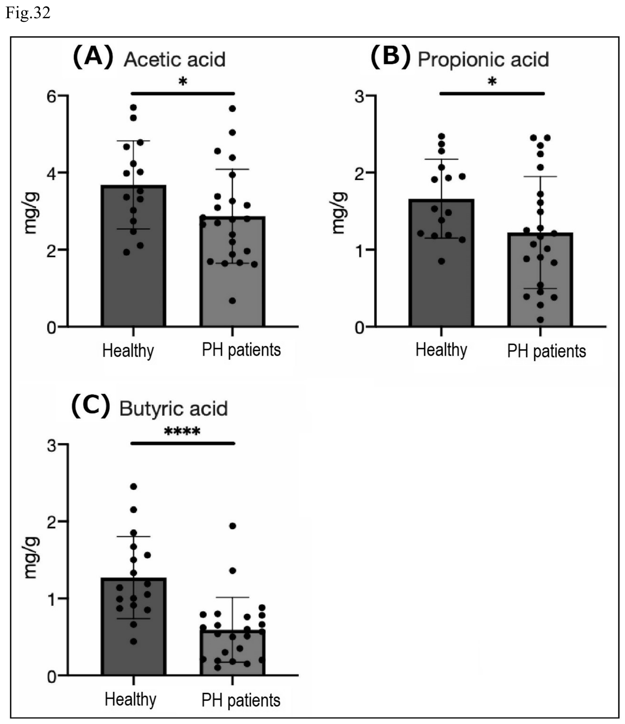

The results are shown in Figs. 32(A) to 32(C). Fig. 32(A) shows the acetic acid levels, Fig. 32(B) shows the propionic acid levels, and Fig. 32(C) shows the butyric acid levels. The acetic acid, propionic acid and butyric acid levels in the feces of the PH patients were significantly decreased compared to those in the feces of the healthy subjects.

### Example 6: Improvement of pulmonary hypertension by administration of short-chain fatty acids

Six-week-old male SD rats (Oriental Yeast) were housed continuously in a hypoxic chamber at 10% oxygen for three weeks to prepare a rat model of pulmonary hypertension induced by exposure to hypoxia in the same manner as in Example 1. From the start of exposure to hypoxia, the rats were orally given water containing 100 mM butyrate or vehicle ad libitum for three weeks. The butyrate-containing water was prepared by dissolving sodium butyrate (SIGMA) in distilled water at a concentration of 100 mM. The right ventricular systolic pressure and the right ventricular hypertrophy were determined for the rats at the age of 9 weeks. Measurement of the right ventricular systolic pressure was performed in the same manner as in the section (2) in A. Experimental materials and methods in Example 1. Measurement of the right ventricular hypertrophy was performed in the same manner as in the section (3) Ratio of right ventricle to left ventricle weight in A. Experimental materials and methods.

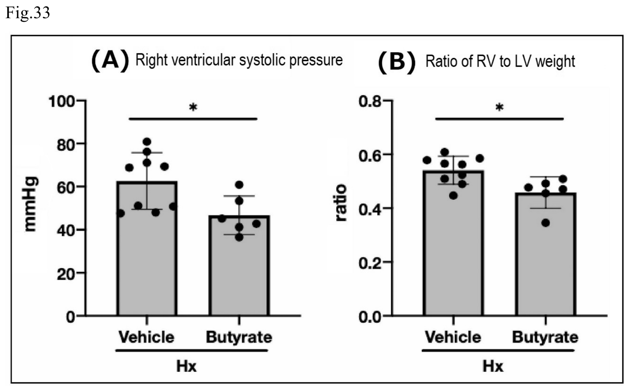

The results are shown in Figs. 33(A) and 33(B). Fig. 33(A) shows the right ventricular systolic pressure, and Fig. 33(B) shows the ratio of RV to LV weight (the right ventricular hypertrophy). The butyrate administration group (Butyrate) showed significant inhibition of the right ventricular systolic pressure and the right ventricular hypertrophy compared to the vehicle administration group (Vehicle). The results from Examples 5 and 6 demonstrate that PH patients have significantly decreased levels of short-chain fatty acids in the feces compared to those in the feces of healthy subjects, suggesting that oral administration of short-chain fatty acids may improve the phenotypes of pulmonary hypertension.

The present invention is not limited to each of the embodiments and Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

## Claims

1. A composition for use in improving pulmonary hypertension, wherein the composition comprises at least one substance capable of normalizing gut microbiota in a patient with pulmonary hypertension as an active ingredient, wherein the substance is a substance capable of reducing gut bacteria that are increased in a patient with pulmonary hypertension compared to those in a healthy subject, wherein the gut bacteria that are increased in a patient with pulmonary hypertension are bacteria belonging to the genus *Actinomyces, Rothia, Citrobacter, Veillonella, Escherichia, Gemella, Granulicatella, Atopobium, Enterobacter, Streptococcus, Abiotrophia, Klebsiella, Cronobacter, Shigella, Salmonella, Sutterella* or *Haemophilus.*

2. The composition for use according to claim 1, wherein the bacteria belonging to the genus *Streptococcus* are *Streptococcus infantis, Streptococcus parasanguinis, Streptococcus mitis, Streptococcus oralis, Streptococcus pneumoniae* or *Streptococcus salivarius,* wherein the bacteria belonging to the genus *Sutterella* is *Sutterella wadsworthensis,* wherein the bacteria belonging to the genus *Klebsiella* is *Klebsiella pneumoniae,* wherein the bacteria belonging to the genus *Rothia* is *Rothia mucilaginosa,* wherein the bacteria belonging to the genus *Haemophilus* is *Haemophilus parainfluenzae,* or wherein the bacteria belonging to the genus *Veillonella* are *Veillonella parvula* or *Veillonella* unclassified.

3. The composition for use according to claim 2, wherein the substance is an agent having antibacterial activity against normal flora bacteria in an oral cavity, and wherein the normal flora bacteria in an oral cavity are *Streptococcus infantis, Streptococcus parasanguinis, Klebsiella pneumoniae, Rothia mucilaginosa, Streptococcus mitis, Streptococcus oralis, Streptococcus pneumoniae, Streptococcus salivarius, Veillonella parvula* or *Veillonella* unclassified, and wherein the agent having antibacterial activity against normal flora bacteria in an oral cavity is a penicillin, cephem, penem, carbapenem, macrolide, lincomycin, ketolide, newquinolone, glycopeptide, streptogramin, tetracycline, chloramphenicol, quinolone, peptide, aminoglycoside, monobactam, nitroimidazole or fosfomycin antibiotic.

4. A composition for use in improving pulmonary hypertension, wherein the composition comprises at least one substance capable of normalizing gut microbiota in a patient with pulmonary hypertension as an active ingredient, wherein the substance is a substance capable of increasing gut bacteria that are decreased in a patient with pulmonary hypertension compared to those in a healthy subject, wherein the gut bacteria that are decreased in a patient with pulmonary hypertension are bacteria belonging to the genus *Butyricimonas, Alistipes, Adlercreutzia, Acidaminococcus, Sutterella, Oscillospira, Rikenella, Lachnospira, Holdemania, Eubacterium, Subdoligranulum, Bilophila, Bifidobacterium, Parasutterella, Roseburia, Faecalibacterium, Parabacteroides, Megamonas, Phascolarctobacterium, Agathobacter, Hydrogenoanaerobacterium, Blautia, Ruminiclostridium, Paraprevotella, Acetanaerobacterium, Dorea, Fusicatenibacter, Coprococcus, Gordonibacter, Coprobacter, Oscillibacter, Anaerostipes* or *Bacteroides.*

5. The composition for use according to claim 4, wherein the bacteria belonging to the genus *Eubacterium* are *Eubacterium hallii, Eubacterium ventriosum,* or *Eubacterium eligens,* wherein the bacteria belonging to the genus *Bilophila* is *Bilophila* unclassified, wherein the bacteria belonging to the genus *Bifidobacterium* are *Bifidobacterium adolescentis* or *Bifidobacterium pseudocatenulatum,* wherein the bacteria belonging to the genus *Parasutterella* is *Parasutterella excrementihominis,* wherein the bacteria belonging to the genus *Roseburia* are *Roseburia hominis* or *Roseburia intestinalis,* wherein the bacteria belonging to the genus *Alistipes* are *Alistipes onderdonkii* or *Alistipes* sp. AP11, wherein the bacteria belonging to the genus *Faecalibacterium* is *Faecalibacterium prausnitzii,* wherein the bacteria belonging to the genus *Parabacteroides* is *Parabacteroides johnsonii,* wherein the bacteria belonging to the genus *Bacteroides* are *Bacteroides cellulosilyticus, Bacteroides uniformis* or Bacteroidales bacterium ph8, wherein the bacteria belonging to the genus *Subdoligranulum* is *Subdoligranulum sp. 4_3_54A2FAA,* or wherein the bacteria belonging to the genus *Lachnospira* is Lachnospiraceae bacterium 1_1_57FAA.

6. A method for predicting the prognosis of a patient with pulmonary hypertension, the method comprising (1) detecting one or more types of bacteria selected from bacteria belonging to the genus *Rothia, Streptococcus, Haemophilus, Veillonella* or *Lactobacillus* in gut microbiota DNA extracted from feces of the patient with pulmonary hypertension, and (2) determining that the patient with pulmonary hypertension has a poor prognosis when the one or more types of bacteria are detected.

7. The method according to claim 6, wherein the bacteria belonging to the genus *Rothia* are *Rothia mucilaginosa, Rothia dentocariosa,* or *Rothia aeria,* wherein the bacteria belonging to the genus *Streptococcus* are *Streptococcus salivarius, Streptococcus parasanguinis, Streptococcus vestibularis, Streptococcus oligofermentans, Streptococcus anginosus, Streptococcus mitis, Streptococcus oralis, Streptococcus pneumoniae, Streptococcus mutans, Streptococcus intermedius, Streptococcus peroris,* or *Streptococcus tigurinus,* wherein the bacteria belonging to the genus *Haemophilus* is *Haemophilus parainfluenzae,* wherein the bacteria belonging to the genus *Veillonella* are *Veillonella parvula, Veillonella atypica, Veillonella disper, Veillonella denticariosi,* or *Veillonella rogosae,* wherein the bacteria belonging to the genus *Lactobacillus* are *Lactobacillus delbrueckii, Lactobacillus acidophilus, Lactobacillus crispatus, Lactobacillus gasseri, Lactobacillus helveticus, Lactobacillus johnsonii, Lactobacillus kefiranofaciens, Lactobacillus paragasseri, Lactobacillus casei, Lactobacillus paracasei, Lactobacillus rhamnosus, Lactobacillus sakei, Lactobacillus curvatus, Lactobacillus salivarius, Lactobacillus plantarum, Lactobacillus pentosus, Lactobacillus fermentum, Lactobacillus reuteri, Lactobacillus brevis, Lactobacillus fructivorans, Lactobacillus lindneri, Lactobacillus sanfranciscensis,* or *Lactobacillus kunkeei.*
